# FASCICULE DE BREVET EUROPEEN

(11) **EP 1 675 836 B1**
(45) Date de publication et mention de la délivrance du brevet: **13.04.2016**
(21) Numéro de dépôt: 04791489.0
(22) Date de dépôt: 08.10.2004
(51) Int. Cl.: C07D 277/28, A61K 31/426, A61P 25/28, A61P 29/00, A61P 25/08

(54) **DERIVES DU 4-PHENYLTHIAZOLE ET LEUR UTILISATION COMME MEDICAMENTS POUR LE TRAITEMENT DE MALADIES NEURODEGENERATIVES, LA DOULEUR ET L'EPILEPSIE**
DERIVATE VON 4-PHENYLTHIAZOLEN UND DEREN VERWENDUNG ALS MEDIKAMENTE ZUR BEHANDLUNG VON NEURODEGENERATIVEN ERKRANKUNGEN, SCHMERZEN UND EPILEPSIE
DERIVATIVES OF 4-PHENYLTHIAZOLES AND THE USE THEREOF AS MEDICAMENTS FOR TREATING NEURODEGENERATIVE DISEASES, PAIN AND EPILEPSY

(30) Priorité: 08.10.2003 US 681002; 10.08.2004 US 915001
(43) Date de publication de la demande: 05.07.2006
(73) Titulaire: IPSEN PHARMA, 92100 Boulogne-Billancourt (FR)
(72) Inventeur: CHABRIER DE LASSAUNIERE, Pierre-Etienne, F-75016 Paris (FR); HARNETT, Jeremiah, F-91190 Gif-sur-Yvette (FR); BIGG, Dennis, F-91190 Gif-sur-Yvette (FR); LIBERATORE, Anne-Marie, F-78610 Auffargis (FR); THURIEAU, Christophe, F-75116 Paris (FR); DONG, Zheng, Xin, Holliston, MA 01746 (US)
(74) Mandataire: Audonnet, Nathalie
(86) Numéro de dépôt international: PCT/FR2004/002537
(87) Numéro de publication internationale: WO 2005/035510

(56) Documents cités:
- WO-A-03/009843
- WO-A2-01/26656
- WO-A2-02/083656
- WO-A2-2004/011402

## Description

La présente invention concerne de nouveaux dérivés du 4-phénylthiazole et du 4-phénylimidazole et leur utilisation en tant que médicaments. Lesdits médicaments sont destinés à inhiber les monoamine oxydases (MAO) et/ou la peroxydation lipidique et/ou à agir en tant que modulateurs des canaux sodiques.

Les composés de l'invention présentent souvent 2 ou 3 des activités citées ci-dessus, lesquelles leur confèrent des propriétés pharmacologiques avantageuses.

En effet, compte tenu du rôle potentiel des MAO et des ROS (« *reactive oxygen species* » ou espèces réactives de l'oxygène, à l'origine de la peroxydation lipidique) en physiopathologie, les nouveaux dérivés de la présente invention peuvent produire des effets bénéfiques ou favorables dans le traitement de pathologies où ces enzymes et/ou ces espèces radicalaires sont impliquées. Notamment :
- les troubles du système nerveux central ou périphérique comme par exemple les maladies neurologiques où l'on peut notamment citer la maladie de Parkinson, les traumatismes cérébraux ou de la moelle épinière, l'infarctus cérébral, l'hémorragie sub arachnoïde, l'épilepsie, le vieillissement, les démences séniles, la maladie d'Alzheimer, la chorée de Huntington, la sclérose latérale amyotrophique, les neuropathies périphériques, la douleur ;
- la schizophrénie, les dépressions, les psychoses ;
- les troubles de la mémoire et de l'humeur ;
- les pathologies comme par exemple la migraine ;
- les troubles du comportement, la boulimie et l'anorexie ;
- les maladies auto-immunes et virales comme par exemple le lupus, le sida, les infections parasitaires et virales, le diabète et ses complications, la sclérose en plaques.
- l'addiction aux substances toxiques ;
- les pathologies inflammatoires et prolifératives ;
- et plus généralement toutes les pathologies caractérisées par une production excessive des ROS et/ou une participation des MAO.

Dans l'ensemble de ces pathologies, il existe des évidences expérimentales démontrant l'implication des ROS (Free Radic. Biol. Med. (1996) 20, 675-705 ; Antioxid. Health. Dis. (1997) 4 (Handbook of Synthetic Antioxidants), 1-52) ainsi que l'implication des MAO (Goodman & Gilman's : The pharmacological basis of therapeutics, 9th ed., 1995,431-519).

L'intérêt d'une combinaison des activités inhibitrice de MAO et inhibitrice de la peroxydation lipidique est par exemple bien illustré dans la maladie de Parkinson. Cette pathologie est caractérisée par une perte des neurones dopaminergiques de la voie nigrostriatal dont la cause serait en partie liée à un stress oxydatif dû aux ROS. De la dopamine exogène à partir de L Dopa est utilisé en thérapeutique afin de maintenir des taux suffisants de dopamine. Les inhibiteurs de MAO sont aussi utilisés avec la L Dopa pour éviter sa dégradation métabolique mais n'agissent pas sur les ROS. Des composés agissant à la fois sur les MAO et les ROS auront donc un avantage certain.

Par ailleurs, le caractère de modulateur des canaux sodiques est très utile pour des indications thérapeutiques comme :
- le traitement ou la prévention de la douleur, et notamment :
   ❖ des douleurs post-opératoires,
   ❖ de la migraine,
   ❖ des douleurs neuropathiques telles que la névralgie du trijumeau, la douleur post-herpétique, les neuropathies diabétiques, les névralgies glosso-pharyngiennes, les radiculopathies et neuropathies secondaires à des infiltrations métastatiques, l'adiposis dolorosa et les douleurs liées aux brûlures,
   ❖ des douleurs centrales consécutives aux accidents cérébraux vasculaires, lésions thalamiques et sclérose en plaques, et
   ❖ des douleurs chroniques inflammatoires ou liées à un cancer ;
- le traitement de l'épilepsie ;
- le traitement de troubles liés à la neurodégénération, et en particulier :
   ❖ des accidents cérébraux vasculaires,
   ❖ du traumatisme cérébral, et
   ❖ de maladies neurodégénératives telles que la maladie d'Alzheimer, la maladie de Parkinson et la sclérose latérale amyotrophique ;
- le traitement des troubles bipolaires et du syndrome du colon irritable.

Les avantages concrets de la présence dans un composé d'au moins une de ces activités ressortent donc clairement de ce qui précède.

La demande de brevet européen EP 432 740 décrit des dérivés d'hydroxyphénylthiazoles, lesquels peuvent être utilisés dans le traitement des maladies inflammatoires, en particulier les maladies rhumatismales. Ces dérivés d'hydroxyphénylthiazoles montrent des propriétés de piégeurs de radicaux libres et d'inhibiteurs du métabolisme de l'acide arachidonique (ils inhibent la lipoxygénase et la cyclooxygénase).

D'autres dérivés d'hydroxyphénylthiazoles ou d'hydroxyphényloxazoles sont décrits dans la demande de brevet PCT WO 99/09829. Ceux-ci possèdent des propriétés analgésiques.

Un certain nombre de dérivés du 4-phénylimidazole ont par ailleurs été décrits par la demanderesse dans la demande de brevet PCT WO 99/64401 comme agonistes ou antagonistes de la somatostatine. Lesdits dérivés d'imidazoles possèdent cependant des propriétés thérapeutiques dans des domaines différents de ceux indiqués ci-dessus (la suppression de l'hormone de croissance et le traitement de l'acromégalie, le traitement de la resténose, l'inhibition de la sécrétion d'acide gastrique et la prévention des saignements gastro-intestinaux notamment).

Par ailleurs, les composés de formule générale **(A1)** 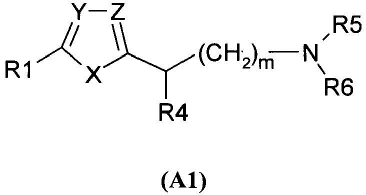 dans laquelle
R1 représente l'un des radicaux aryle, hétéroaryle, aralkyle ou cycloalkyle optionnellement substitués par un à trois substituants choisis indépendamment parmi un atome halogène, le radical CF₃, CN, OH, alkyle ou alkoxy, SO₂R9 avec R9 représentant NH₂ ou NHCH₃ ;
X représente NR2, R2 représentant H ou alkyle ;
Y représente N ou CR3 ;
Z représente CR3 ou N ;
à la condition toutefois que Y et Z ne sont pas tous deux CR3 ou N en même temps ;
R3 représente H, alkyle, halogène, hydroxyalkyle ou phényle éventuellement substitué par 1 à 3 subtituants choisis parmi H, CF₃, CN, SO₂NH₂, OH, alkyle ou alkoxy ;
m représente 0, 1 ou 2 ;
R4 représente H ou alkyle ;
lorsque Z représente CR3, alors R3 et R4 peuvent aussi représenter ensemble -(CH₂)ₙ₁- avec n1 entier de 2 à 4 ou R2 et R4 peuvent aussi représenter ensemble -(CH₂)ₙ₂- avec n2 entier de 2 à 4 ;
R5 et R6 représentent indépendamment H, alkyle, alkoxy, aryle ou aralkyle ;
NR5R6 pouvant aussi représenter ensemble (notamment) :
   - le radical 2-(1,2,3,4-tétrahydroquinolyl) éventuellement substitué,
   - un radical 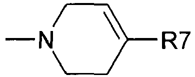 dans lequel R7 représente l'un des radicaux phényle, benzyle ou phénéthyle dans lequels le cycle phényle peut être substitué ;
   - un radical 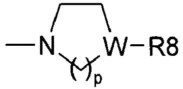
      dans lequel p est un entier de 1 à 3,
      W est N et R8 représente H, CF₃, l'un des radicaux phényle, pyridyle ou pyrimidinyle éventuellement substitués de 1 à 2 fois par des radicaux choisis parmi halogène, OH, alkyle ou alkoxy, ou
      W est CH et R8 représente phényle éventuellement substitué ou aralkyle éventuellement substitué sur le groupe aryle ;
ont été décrits dans la demande de brevet PCT WO 96/16040 en tant qu'agonistes partiels ou antagonistes des sous-récepteurs de la dopamine du cerveau ou en tant que formes prodrogues de tels agonistes partiels ou antagonistes. Ces composés présenteraient de ce fait des propriétés intéressantes dans le diagnostic et le traitement de désordres affectifs tels que la schizophrénie et la dépression ainsi que certains désordres du mouvement tels que la maladie de Parkinson.

Il a également été décrit dans la demande de brevet PCT WO 98/27108 que certains amides de formule générale (A2) 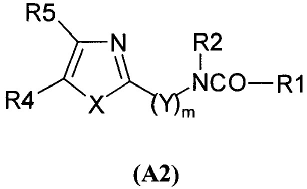 dans laquelle :
R1 représente notamment un radical alkyle, phényle éventuellement substitué ou aryle hétérocyclique éventuellement substitué ;
R2 représente H ou phénylalkyle ;
R4 représente H, quinolyle, 3-4-méthylènedioxyphényle ou l'un des radicaux phényle ou pyridyle éventuellement substitués, par un ou des radicaux choisis notamment parmi alkyle, alkoxy, alkylthio, hydroxy éventuellement protégé, amino, alkylamino, dialkylamino ;
R5 représente H ou un radical imidazolyle, phényle, nitrophényle, phénylalkyle, ou encore un radical -CO-N(R7)(R8), dans lequel R7 et R8 représentent indépendamment H, phényle, phénylalkyle, alkyle ou alkoxy ;
ou R4 et R5 en combinaison forment un groupe de formule -CH=CH-CH=CH- ;
Y est un radical phénylène substitué par un radical phényle, phénoxy ou phénylalkoxy, ou un groupe de formule -CH(R3)-, dans laquelle R3 représente H ou un radical de formule -(CH₂)ₙ-R6, dans laquelle R6 représente un radical hydroxy éventuellement protégé, acyle, carboxy, acylamino, alkoxy, phénylalkoxy, alkylthio, phényle éventuellement substitué, pyridyle éventuellement substitué, pyrazinyle, pyrimidinyle, furyle, imidazolyle, naphtyle, N-alkylindolyle ou 3,4-méthylènedioxyphényle et n est un entier de 0 à 3 ;
R2 et R3 pris ensemble avec les atomes de carbone qui les portent pouvant former un groupe phényle ;
X représente S ou NR9 ;
R9 représentant H, un radical alkyle ou cycloalkyle, ou encore un radical benzyle éventuellement substitué une fois sur sa partie phényle par H, alkyle ou alkoxy ;
sont des inhibiteurs des NO synthases et peuvent être utilisés pour traiter des maladies qui comprennent notamment l'ischémie cardiovasculaire ou cérébrale, l'hémorragie cérébrale, les troubles du système nerveux central, la maladie d'Alzheimer, la sclérose en plaques, le diabète, l'hépatite, la migraine, l'arthrite rhumatoïde et l'ostéoporose.

La demanderesse a aussi décrit dans la demande de brevet PCT WO 01/26656 une famille de composés comprenant des dérivés du 4-phénylthiazole et du 4-phénylimidazole. Les composés de cette famille inhibent les monoamine oxydases (MAO) et/ou la peroxydation lipidique et/ou agissent en tant que modulateurs des canaux sodiques.

La demanderesse a maintenant découvert de façon surprenante que certains composés particuliers appartenant à la famille décrite dans la demande de brevet PCT WO 01/26656, à savoir les composés décrits dans les exemples de la présente demande, possédaient des propriétés particulièrement avantageuses par rapport à leurs homologues les plus proches de la demande de brevet PCT WO 01/26656.

Ces propriétés avantageuses rendent ces composés particulièrement adaptés à une utilisation dans le traitement des maladies neurodégénératives, et notamment celles indiquées précédemment, de la douleur (particulièrement la douleur d'origine neuropathique) ou de l'épilepsie.

Il est donc divulgué l'utilisation d'un des composés suivants :
- 2-[4-(4-aminophényl)-1*H*-imidazol-2-yl]éthylcarbamate de butyle ;
- *N*,2-diméthyl-1-[4-(10H-phénothiazin-2-yl)-1,3-thiazol-2-yl]propan-1-amine ;
- *N*,2-diméthyl-l-[4-(10H-phénoxazin-2-yl)-l,3-thiazol-2-yl]propan-l-aminé ;
- *N*,3-diméthyl-1-[4-(10H-phénoxazin-2-yl)-1,3-thiazol-2-yl]butan-1-amine ;
- *N*,3-diméthyl-1-[4-(10H-phénothiazin-2-yl)-1,3-thiazol-2-yl]butan-1-amine ;
- 2,6-di-*tert*-butyl-4-{2-[3-méthyl-1-(méthylamino)butyl]-1,3-thiazol-4-yl}phénol ;
- [4-(3,5-di-*tert*-butylphényl)-1,3-thiazol-2-yl]méthylamine ;
- 2,6-di-*tert*-butyl-4-{2-[(1S)-1-(méthylamino)éthyl]-1,3-thiazol-4-yl}phénol ;
- 2,6-di-*tert*-butyl-4-{2-[(1R)-1-(méthylamino)éthyl]-1,3-thiazol-4-yl}phénol ;
- *N*-{[4-(3,5-di-*tert*-butylphényl)-1,3-thiazol-2-yl]méthyl}-N-méthylamine ;
- *N*-méthyl-*N*-{[4-(3,4,5-triméthoxyphényl)-1,3-thiazol-2-yl]méthyl}amine;
- *N*-{[4-(3,5-di-tert-butyl-4-hydroxyphényl)-1,3-thiazol-2-yl]méthyl}glycinate d'éthyle ;
- *N*-{[4-(3,5-di-*tert*-butyl-4-hydroxyphényl)-1,3-thiazol-2-yl]méthyl}glycine ;
- 2,6-di-*tert*-butyl-4-{2-[(4-méthoxypipéridin-1-yl)méthyl]-1,3-thiazol-4-yl}phénol ;
- *N*-méthyl-*N*-{(*1S*)-2-méthyl-1-[4-(10H-phénothiazin-2-yl)-1,3-thiazol-2-yl]propyl}amine ;
- *N*,2-diméthyl-1-[4-(10-méthyl-10*H*-phénothiazin-2-yl)-1,3-thiazol-2-yl]propan-1-amine ;
- *N*-méthyl-*N*-{(*1S*)-2-méthyl-1-[4-(10*H*-phénoxazin-2-yl)-1,3-thiazol-2-yl]propyl}amine ;
- 4-{2-[(1*R*)-1-aminoéthyl]-1,3-thiazol-4-yl}-2,6-di-*tert*-butylphénol ;
- 4-{2-[(1,*S*)-1-aminoéthyl]-1,3-thiazol-4-yl}-2,6-di-*tert*-butylphénol ;
- 4-[2-(1-aminocyclopropyl)-1,3-thiazol-4-yl]-2,6-di-*tert*-butylphénol ;
- 4-{2-[(méthylamino)méthyl]-1,3-thiazol-4-yl}benzène-1,2-diol ;
- N-méthyl-N-{(1R)-2-méthyl-1-[4-(10H-phénothiazin-2-yl)-1,3-thiazol-2-yl]propyl}amine ;
- (*1R*)-2-méthyl-1-[4-(10H-phénothiazin-2-yl)-1,3-thiazol-2-yl]propan-1-amine ;
- *N*-méthyl-*N*-{(*1R*)-2-méthyl-1-[4-(10H-phénoxazin-2-yl)-1,3-thiazol-2-yl]propyl}amine ;
- *N*²-{[4-(3,5-di-*tert*-butyl-4-hydroxyphényl)-1,3-thiazol-2-yl]méthyl}glycinamide ;
- *N*-{[4-(3,5-di-*tert*-butyl-4-hydroxyphényl)-1,3-thiazol-2-yl]méthyl}-*N*-(2-éthoxy-2-oxoéthyl)glycinate d'éthyle ;
- 4-(3,5-di-*tert*-butyl-4-méthoxyphényl)-2-(méthoxyméthyl)-1,3-thiazole ;
- 4-[2-(1-aminocyclopentyl)-1,3-thiazol-4-yl]-2,6-di-tert-butylphénol ;
ou d'un sel pharmaceutiquement acceptable d'un de ces derniers ;
pour préparer un médicament destiné à inhiber les monoamine oxydases (MAO) et/ou la peroxydation lipidique et/ou à agir en tant que modulateurs des canaux sodiques.

Il est aussi divulgué aussi l'utilisation d'un des composés ou sels pharmaceutiquement acceptables décrits ci-dessus pour préparer un médicament destiné à traiter des désordres / pathologies choisis parmi les maladies neurodégénératives, la douleur (particulièrement la douleur d'origine neuropathique) et l'épilepsie.

De préférence, les maladies neurodégénératives destinées à être traitées par un médicament préparé selon l'invention seront choisies parmi la maladie de Parkinson, la maladie d'Alzheimer, la chorée de Huntington et la sclérose latérale amyotrophique. Selon une première variante de l'invention, les composés des exemples selon l'invention (parfois décrits sous forme de sels), ou leurs sels pharmaceutiquement acceptables, seront préférés lorsqu'une activité activité inhibitrice des MAO et/ou des ROS sera recherchée en premier lieu. Plus préférentiellement, composés des exemples 6, et 28 (décrits sous forme de sels chlorhydrate), ou leurs sels pharmaceutiquement acceptables, seront utilisés pour préparer un médicament selon l'invention lorsqu'une activité activité inhibitrice des MAO et/ou des ROS sera recherchée en premier lieu. Encore plus préférentiellement, le composé de l'exemple 28 (décrit sous forme de son sel chlorhydrate) et ses sels pharmaceutiquement acceptables seront utilisés pour préparer un médicament selon l'invention lorsqu'une activité activité inhibitrice des MAO et/ou des ROS sera recherchée en premier lieu.

Selon une autre variante de l'invention, les composés des exemples 8 et 9 (parfois décrits sous forme de sels), ou leurs sels pharmaceutiquement acceptables, seront préférés lorsqu'une activité activité modulatrice des canaux sodiques sera recherchée en premier lieu.

L'invention a également pour objet, à titre de médicaments, les composés suivants :
- 2,6-di-*tert*-butyl-4-{2-[3-méthyl-1-(méthylamino)butyl]-1,3-thiazol-4-yl}phénol ;
- 2,6-di-*tert*-butyl-4-{2-[(1S)-1-(méthylamino)éthyl]-1,3-thiazol-4-yl}phénol ;
- 2,6-di-*tert*-butyl-4-{2-[(*1R*)-1-(méthylamino)éthyl]-1,3-thiazol-4-yl}phénol ;
- 4-{2-[(*1R*)-1-aminoéthyl]-1,3-thiazol-4-yl}-2,6-di-*tert*-butylphénol ;
- 4-{2-[(*1S*)-1-aminoéthyl]-1,3-thiazol-4-yl}-2,6-di-*tert*-butylphénol ;
- 4-[2-(1-aminocyclopropyl)-1,3-thiazol-4-yl]-2,6-di-*tert*-butylphénol ;
- 4-[2-(1-aminocyclopentyl)-1,3-thiazol-4-yl]-2,6-di-tert-butylphénol ;
et les sels pharmaceutiquement acceptables de ces derniers.

L'invention concerne également des compositions pharmaceutiques contenant, à titre de principe actif, au moins l'un des composés suivants :
- 2,6-di-*tert*-butyl-4-{2-[3-méthyl-1-(méthylamino)butyl]-1,3-thiazol-4-yl}phénol ;
- 2,6-di-tert-butyl-4-{2-[(1S)-1-(méthylamino)éthyl]-1,3-thiazol-4-yl}phénol ;
- 2,6-di-*tert*-butyl-4-{2-[(*1R*)-1-(méthylamino)éthyl]-1,3-thiazol-4-yl}phénol ;
- 4-{2-[(*1R*)-1-aminoéthyl]-1,3-thiazol-4-yl}-2,6-di-*tert*-butylphénol ;
- 4-{2-[(*1S*)-1-aminoéthyl]-1,3-thiazol-4-yl}-2,6-di-*tert*-butylphénol ;
- 4-[2-(1-aminocyclopropyl)-1,3-thiazol-4-yl]-2,6-di-*tert*-butylphénol ;
- 4-[2-(1-aminocyclopentyl)-1,3-thiazol-4-yl]-2,6-di-tert-butylphénol ;
ou un sel pharmaceutiquement acceptable d'un de ces composés.

L'invention concerne en outre, à titre de produits industriels nouveaux, les composés suivants :
- 2,6-di-*tert*-butyl-4-{2-[3-méthyl-1-(méthylamino)butyl]-1,3-thiazol-4-yl}phénol ;
- 2,6-di-*tert*-butyl-4-{2-[(1S)-1-(méthylamino)éthyl]-1,3-thiazol-4-yl}phénol ;
- 2,6-di-*tert*-butyl-4-{2-[(*1R*)-1-(méthylamino)éthyl]-1,3-thiazol-4-yl}phénol ;
- 4-{2-[(*1R*)-1-aminoéthyl]-1,3-thiazol-4-yl}-2,6-di-*tert*-butylphénol ;
- 4-{2-[(*1S*)-1-aminoéthyl]-1,3-thiazol-4-yl}-2,6-di-*tert*-butylphénol ;
- 4-[2-(1-aminocyclopropyl)-1,3-thiazol-4-yl]-2,6-di-*tert*-butylphénol ;
- 4-[2-(1-aminocyclopentyl)-1,3-thiazol-4-yl]-2,6-di-tert-butylphénol ;
et les sel de ces composés.

Dans certains cas, les composés selon la présente invention peuvent comporter des atomes de carbone asymétriques. Par conséquent, les composés selon la présente invention ont deux formes énantiomères possibles, c'est-à-dire les configurations "R" et "S". La présente invention inclut les deux formes énantiomères et toutes combinaisons de ces formes, y compris les mélanges racémiques "RS". Dans un souci de simplicité, lorsqu'aucune configuration spécifique n'est indiquée dans les formules de structure ou les noms, il faut comprendre que les deux formes énantiomères et leurs mélanges sont représentés.

Par sel on entend notamment dans la présente demande les sels avec des acides organiques ou inorganiques ainsi que les sels formés à l'aide de bases.

Par sel pharmaceutiquement acceptable, on entend notamment des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt selection for basic drugs", Int. J. Pharm. (1986), 33, 201-217.

Les compositions pharmaceutiques selon la présente invention peuvent être sous forme de solides, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Les compositions pharmaceutiques contenant un composé de l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un médicament selon l'invention pourra se faire par voie topique, orale, parentérale, par injection intramusculaire, etc.

La dose d'administration envisagée pour médicament selon l'invention est comprise entre 0,1 mg à 10 g suivant le type de composé actif utilisé.

Conformément à l'invention, on peut préparer les composés de l'invention par les procédés décrits ci-dessous.

### PREPARATION DES COMPOSES DE L'INVENTION :

Les composés de la présente invention peuvent tous être préparés selon des techniques décrites dans la publication PCT WO 01/26656.

A moins qu'ils ne soient définis d'une autre manière, tous les termes techniques et scientifiques utilisés ici ont la même signification que celle couramment comprise par un spécialiste ordinaire du domaine auquel appartient cette invention. De même, toutes les publications, demandes de brevets, tous les brevets et toutes autres références mentionnées ici sont incorporées par référence.

Les exemples suivants sont présentés pour illustrer les procédures ci-dessus et ne doivent en aucun cas être considérés comme une limite à la portée de l'invention.

### EXEMPLES

### Préparation 1 : 4-[3,5-bis(1,1-diméthyléthyl)-4-hydroxyphényl]-N-méthyl-2-thiazoleméthanamine :

### Étape 1 : N-Boc-sarcosinamide :

15,0 g (0,120 mol) de chlorhydrate de sarcosinamide (N-Me-Gly-NH₂.HCl) sont mis en solution dans du dichlorométhane contenant 46,2 ml (0,265 mol) de diisopropyléthylamine. Le mélange est refroidi à 0 °C puis on ajoute par fractions Boc-O-Boc (28,8 g ; 0,132 mol) et on laisse agiter le mélange une nuit à température ambiante. Le milieu réactionnel est alors versé sur de l'eau glacée et extrait au dichlorométhane. La phase organique est lavée successivement avec une solution aqueuse de bicarbonate de sodium à 10% et à l'eau, puis enfin avec une solution de chlorure de sodium saturée. La phase organique est ensuite séchée sur sulfate de magnésium, filtrée et concentrée sous vide. Le produit obtenu est purifié par cristallisation dans de l'éther diisopropylique pour conduire à un solide blanc avec un rendement de 72%. Point de fusion : 103 °C.

### Étape 2 : 2-{[(1,1-diméthyléthoxy)carbonyl]méthyl}amino-éthanethioamide :

16,0 g (0,085 mol) de l'intermédiaire obtenu à l'étape 1 sont dissous dans du diméthoxyéthane (500 ml) et la solution obtenue est refroidie à 5 °C. Du bicarbonate de sodium (28,5 g ; 0,34 mol) puis, par petites portions, (P₂S₅)₂ (38,76 g ; 0,17 mol) sont ajoutés. Le milieu réactionnel est laissé revenir à température ambiante tout en étant agité durant 24 heures. Après évaporation sous vide des solvants, on ajoute au résidu une solution aqueuse de bicarbonate de sodium à 10% et la solution est extraite à l'aide d'acétate d'éthyle. La phase organique est lavée successivement avec une solution aqueuse de bicarbonate de sodium à 10% et à l'eau, puis enfin avec une solution de chlorure de sodium saturée. La phase organique est ensuite séchée sur sulfate de magnésium, filtrée et concentrée sous vide. Le produit obtenu est purifié par cristallisation dans de l'éther pour conduire à un solide de couleur blanche avec un rendement de 65%. Point de fusion : 150-151 °C.

### Étape 3 : 4-[3,5-bis(1,1-diméthyléthyl)-4-hydroxyphényl]-N-(1,1-diméthyléthoxy)-carbonyl]-N-méthyl-2-thiazoleméthanamine :

L'intermédiaire obtenu à l'étape 2 (4,3 g ; 2,11 mmol) et de la bromo-1-(3,5-di*tert-*butyl-4-hydroxyphényl)éthanone (6,9g ; 2,11 mmol) sont dissous dans du benzène (75 ml) sous atmosphère d'argon puis le mélange est agité à température ambiante durant 12 heures. Le milieu réactionnel est porté au reflux pendant 4 heures. Après évaporation des solvants, le résidu est dilué avec du dichlorométhane et lavé avec une solution saturée en NaCl. La phase organique est séparée, séchée sur sulfate de magnésium, filtrée et concentrée sous vide. Le produit attendu est obtenu après chromatographie sur une colonne de silice (éluant : 20% d'acétate d'éthyle dans de l'heptane) sous forme d'une huile qui cristallise très lentement au réfrigérateur avec un rendement de 28%. Point de fusion : 126,5-127,3 °C.

### Étape 4 : 4-(3,5-bis(1,1-diméthyléthyl)-4-hydroxyphényl]-N-méthyl-2-thiazoleméthanamine :

A une solution de 2,5 g (5,8 mmol) de l'intermédiaire obtenu à l'étape 3 et de 2 ml (1,6 mmol) de triéthylsilane dans 50 ml de dichlorométhane, on ajoute goutte à goutte, à 0 °C, 2,3 ml (29 mmol) d'acide trifluoroacétique. Après une heure d'agitation, le mélange réactionnel est concentré sous vide et le résidu est dilué dans 100 ml d'acétate d'éthyle et 50 ml d'une solution saturée de NaHCO₃. Après agitation et décantation, la phase organique est séchée sur sulfate de magnésium, filtrée et concentrée sous vide. Le résidu est repris dans de l'heptane pour donner, après séchage, un solide blanc avec un rendement de 73%. Point de fusion : 136 °C.

### Étape 5 : chlorhydrate de 4-[3,5-bis(1,1-diméthyléthyl)-4-hydroxyphényl]-N-méthyl-2-thiazoleméthanamine :

2,0 g (0,602 mmol) de l'intermédiaire obtenu à l'étape 4 sont dissous dans de l'éther anhydre. La solution est refroidie à 0 °C puis on ajoute goutte à goutte 18 ml (1,81 mmol) d'une solution de HCl 1*N* dans l'éther. On laisse le mélange revenir à température ambiante tout en le maintenant agité. Après filtration et séchage sous vide, un solide blanc est récupéré avec un rendement de 92%. Point de fusion : 185,3-186,0 °C.

### Préparation 2 : 2,6-di(tert-butyl)-4-(2-{[méthyl(2-propynyl)aminolméthyl}-1,3-thiazol-4-yl)phénol :

A une solution de 0,5 g (1,5 mmol) du composé de la préparation 1 dans 15 ml d'acétonitrile, on ajoute goutte-à-goutte, à 0 °C, 0,52 ml (3,7 mmol) de triéthylamine et un excès de 0,56 g (7,5 mmol) de chloropropargyle. Après une nuit d'agitation, le mélange réactionnel est concentré sous vide et le résidu est dilué avec du dichlorométhane et 50 ml d'une solution saturée de NaCl. Après agitation et décantation, la phase organique est séparée et séchée sur sulfate de magnésium, filtrée et concentrée sous vide. Le produit attendu est obtenu après chromatographie sur une colonne de silice (éluant : 20% acétate d'éthyle dans de l'heptane). Après évaporation, les fractions pures donnent un solide blanc avec un rendement de 20%. Point de fusion : 210-215 °C.
MH+ = 371,20.

### Préparation 3 : {4-[3,5-di(tert-butyl)-4-hydroxyphényl]-1,3-thiazol-2-yl}méthylcarbamate de benzyle :

Le composé est préparé selon un protocole expérimental décrit dans la demande de brevet WO 98/58934 (voir préparation des intermédiaires 26.1 et 26.2), en utilisant Z-Gly-NH₂ à la place du N-Boc sarcosinamide. Le composé attendu est obtenu sous forme d'une huile jaune pâle avec un rendement de 99%.
MH+ = 453,20.

### Préparation 4 : 4-[2-(aminométhyl)-1,3-thiazol-4-yl]-2,6-di(tert-butyl)phénol :

A une solution de 0,106 g (1,1 mmol) du composé de la préparation 3 dans 10 ml de méthanol on ajoute goutte à goutte 0,1 ml d'une solution d'hydroxyde de potassium à 40%. Après une nuit d'agitation à reflux, le mélange réactionnel est concentré sous vide et le résidu est dilué avec du dichlorométhane et lavé avec une solution HCl 1*N* puis 50 ml d'une solution saturée de NaCl. La phase organique est séparée et séchée sur sulfate de magnésium, filtrée et concentrée sous vide. Le produit attendu est obtenu après chromatographie sur une colonne de silice (éluant : 5% d'éthanol dans du dichlorométhane) sous forme d'une mousse marron avec un rendement de 76%.
MH+ = 319,29.

### Préparation 5 : 2-[3,5-bis(1,1-diméthyléthyl)-4-hydroxyphényl]-4-oxazoleéthanol :

[il s'agit de l'intermédiaire 1.C de la demande PCT WO 99/09829 ; alternativement, ce composé peut aussi être obtenu selon la procédure décrite dans J. Med. Chem. (1996), 39, 237-245.]

### Préparation 6 : 2,6-ditert-butyl-4-(4-{2-[méthyl(2-propynyl)amino]éthyl}-1,3-oxazol-2-yl)phénol :

Le composé de la préparation 5 est transformé en dérivé bromé, intermédiaire 3, selon la procédure indiquée dans le schéma 1(c) de la demande PCT WO 99/09829. Ensuite le dérivé bromé (0,5 g ;1,31 mmol) est ajouté à une solution de N-méthylpropargylamine 0,34 ml (3,94 mmol) et de carbonate de potassium (1,11 g) dans du diméthylformamide (20 ml). Après une nuit d'agitation à 80 °C, le mélange réactionnel est concentré sous vide et le résidu est dilué avec du dichlorométhane et 50 ml d'une solution saturée en NaCl. Après agitation et décantation, la phase organique est séparée et séchée sur sulfate de magnésium, filtrée et concentrée sous vide. Le produit attendu est obtenu après chromatographie sur une colonne de silice (éluant : 50% acétate d'éthyle dans de l'heptane). Après évaporation, les fractions pures donnent une huile jaune avec un rendement de 24%.
MH+ = 369,30.

### Préparation 7 : chlorhydrate de 2,6-ditert-butyl-4-{4-[2-(1-pipérazinyl)éthyl]-1,3-oxazol-2-yl}phénol :

### Étape 1 : tert-butyl 4-{2-[2-(3,5-ditert-butyl-4-hydroxyphényl)-1,3-oxazol-4-yl]éthyl}-1-pipérazinecarboxylate

Le protocole expérimental utilisé est identique à celui décrit pour la préparation 6, le pipérazinecarboxylate de *tert*-butyle étant utilisé comme produit de départ à la place de la N-méthylpropargylamine. On obtient une huile brune avec un rendement de 72%. MH+ = 486,20.

### Étape 2 : chlorhydrate de 2,6-ditert-butyl-4-{4-[2-(1-pipérazinyl)éthyl]-1,3-oxazol-2-yl}phénol

Dans une solution à 0 °C de l'intermédiaire obtenu à l'étape 1 (0,450 g ; 9,27 mmol) dans de l'acétate d'éthyle (30 ml), on passe un courant de HCl gaz bulle à bulle. On laisse le mélange revenir à température ambiante durant la nuit. On fait passer un courant d'argon à travers la masse réactionnelle, puis la poudre obtenue est filtrée et lavée avec de l'acétate d'éthyle puis de l'éther pour conduire à un solide blanc avec un rendement de 70%. Point de fusion : > 200 °C.

### Préparation 8 : chlorhydrate de N-méthyl[4-(10H-phénothiazin-2-yl)-1,3-thiazol-2-yl]méthanamine :

### Étape 1 : 2-chloro-1-(10H-phénothiazin-2-yl)éthanone :

De la 2-bromo-1-[10-(chloroacétyl)-10*H*-phénothiazin-2-yl)éthanone (2,2 g; 5,55 mmol ; préparée d'après un protocole décrit dans J. Heterocyclic. Chem. (1978), 15, 175, suivi par une réaction de Friedel-Crafts) est dissoute à chaud dans un mélange d'acide acétique (20 ml) et de HCl à 20% (5,5 ml) et le mélange obtenu est porté à reflux pendant 30 minutes. Après refroidissement, filtration du précipité, rinçage avec de l'acide acétique (5 ml) et séchage sous vide, le solide obtenu est purifié par cristallisation dans du toluène pour conduire à un produit marron avec un rendement de 82% Point de fusion : 190-191 °C (valeur dans la littérature : 197-198 °C).

### Étape 2 : chlorhydrate de N-méthyl[4-(10H-phénothiazin-2-yl)-1,3-thiazol-2 yl]méthanamine :

L'intermédiaire obtenu à l'étape 1 (0,280 g ; 1,0 mmol) et le 2-amino-2-thioxoéthyl(méthyl)carbamate de *tert*-butyle (0,204 g ; 1,0 mmol ; décrit par exemple dans la demande de brevet PCT WO 98/58934) sont dissous dans du toluène et le mélange est porté à reflux pendant 18 heures. Après évaporation du toluène et refroidissement du mélange réactionnel à 0 °C, ce dernier est repris dans une solution 4*N* de HCl dans du dioxane (10 ml) et le mélange agité une heure à 0 °C avant de laisser la température revenir à température ambiante. Le solide formé est filtré et rincé avec de l'éther. Le produit attendu est obtenu après purification par cristallisation dans de l'acide acétique glacial à chaud pour conduire à un solide verdâtre. Point de fusion : >275 °C.

### Préparation 9 : 2-(4-[1,1'-biphényl]-4-yl-1H-imidazol-2-yl)éthylcarbamate de butyle :

### Étape 1 : N-(butoxycarbonyl)-β-alanine :

Une solution contenant de la β-alanine (8,9 g ; 0,1 mol) et 100 ml d'une solution d'hydroxyde de sodium 1N est refroidie à 10 °C. Du *n*-butyl chloroformate (13,66 g ; 0,1 mol) et 50 ml d'une solution d'hydroxyde de sodium 2 N sont ajoutés simultanément. Après 16 heures d'agitation à 23 °C, on ajoute environ 10 ml d'une solution d'acide chlorhydrique concentré (environ 11 N) pour ajuster le pH à 4-5. L'huile obtenue est extraite à l'acétate d'éthyle (2 x 50 ml), lavée avec de l'eau puis séchée sur sulfate de magnésium. Le produit cristallise dans l'isopentane sous forme d'une poudre blanche (rendement de 68%). Point de fusion : 50,5 °C.

### Étape 2 : 2-(4-[1,1'-biphényl]-4-yl-1H-imidazol-2-yl)éthylcarbamate de butyle :

Un mélange de N-(butoxycarbonyl)-β-alanine (préparée à l'étape 1 ; 5,67 g ; 0,03 mol) et de carbonate de césium (4,89 g ; 0,015 mol) dans 100 ml d'éthanol est agité à 23 °C pendant 1 heure. L'éthanol est éliminé par évaporation sous pression réduite dans un évaporateur rotatif. Le mélange obtenu est dissous dans 100 ml de diméthylformamide puis de la 4-phényl-bromoacétophénone (8,26 g ; 0,03 mol) est ajoutée. Après 16 heures d'agitation, le solvant est évaporé sous pression réduite. Le mélange obtenu est repris dans de l'acétate d'éthyle puis le bromure de césium est filtré. L'acétate d'éthyle du filtrat est évaporé et l'huile réactionnelle est reprise dans un mélange de xylène (100 ml) et d'acétate d'ammonium (46,2 g ; 0,6 mol). On chauffe à reflux environ une heure et demie puis, après refroidissement, un mélange d'eau glacée et d'acétate d'éthyle est versé dans le milieu réactionnel. Après décantation, la phase organique est lavée avec une solution saturée en bicarbonate de sodium, séchée sur sulfate de magnésium puis évaporée sous vide. Le solide obtenu est filtré puis lavé à l'éther pour donner une poudre de couleur beige clair (rendement de 50%). Point de fusion : 136,7 °C.
MH+ = 364,3.

### Préparation 10 : chlorhydrate de 2,5,7,8-tétraméthyl-2-{2-[(méthylamino)méthyl]-1,3-thiazol-4-yl}-6-chromanol

### Étape 1 : 6-hydroxy-N-méthoxy-N,2,5,7,8-pentaméthyl-2-chromanecarboxamide

A une solution de 5,0 g (20,0 mmol) de l'acide (R,S) 6-hydroxy-2,5,7,8-tétraméthyl-2-chromanecarboxylique (Trolox^{®}) dans 175 ml de DMF, on ajoute successivement 2,2 g (22,0 mmol) de chlorhydrate de O,N-diméthylhydroxylamine, de la triéthylamine (6,2 ml), 3,0 g (22,0 mmol) d'hydroxybenzotriazole et 4,2 g (22,0 mmol) de chlorhydrate de 1-(3-diméthylaminopropyl)-3-éthyl-carbodiimide. Après avoir agité le mélange réactionnel une nuit durant à 25 °C, on dilue l'ensemble avec de l'eau glacée et l'agitation est maintenue 30 minutes supplémentaires. Le produit est extrait à l'aide de 3 fois 100 ml d'acétate d'éthyle. La solution organique est lavée successivement avec une solution aqueuse de bicarbonate de sodium à 10%, à l'eau, avec une solution d'acide citrique aqueuse à 10% et finalement avec une solution de chlorure de sodium saturée. La phase organique est ensuite séchée sur sulfate de magnésium, filtrée et concentrée sous vide. Le produit obtenu est purifié par cristallisation dans de l'éther pour conduire à un solide de couleur blanche avec un rendement de 63%. Point de fusion : 139-140 °C.
MH+ = 294.

### Étape 2 : 1-(6-hydroxy-2,5,7,8-tétraméthyl-3,4-dihydro-2H-chromen-2-yl)éthanone

A une solution de 2,93 g (10,0 mmol) de l'intermédiaire obtenu à l'étape 1 dans 100 ml de THF, on ajoute goutte à goutte à la température de -30 °C une solution de méthyllithium (1,6 M ; 31,25 ml ; 50,0 mmol) et on laisse le mélange sous agitation 1 heure à -10 °C. Le milieu réactionnel est hydrolysé avec NH₄Cl en solution aqueuse saturée.Le produit est extrait à l'aide de 3 fois 150 ml d'acétate d'éthyle. La phase organique est finalement lavée avec du chlorure de sodium en solution aqueuse saturée avant d'être ensuite séchée sur sulfate de magnésium, filtrée et concentrée sous vide. Le produit obtenu est purifié par cristallisation dans de l'éther diisopropylique pour conduire à un solide blanc avec un rendement de 80,7%. Point de fusion : 97-98 °C. MH+ = 248.

### Étape 3 : 2-bromo-1-(6-hydroxy-2,5,7,8-tétraméthyl-3,4-dihydro-2H-chromen-2-yl)éthanone

L'intermédiaire obtenu à l'étape 2 (0,777 g ; 3,13 mmol) est dissous dans de l'éthanol (25 ml) sous courant d'argon. La solution est refroidie vers 0 °C et du brome (0,18 ml ; 4,20 mmol) est ajouté en une seule portion (voir J. Am. Chem. Soc. (1999), 121, 24), puis le mélange est agité une demi-heure en laissant la température revenir à température ambiante. L'excès de brome est éliminé par barbotage d'argon puis on laisse le mélange sous agitation durant 2 heures et demie. L'éthanol est évaporé et le produit obtenu est purifié par cristallisation dans le toluène. Après filtration et lavage avec de l'isopentane, on obtient un solide marron avec un rendement de 36%. Point de fusion : décomposition à partir de 125 °C.
MH+ = 326.

### Étape 4 : chlorhydrate de 2,5,7,8-tétraméthyl-2-(2-((méthylamino)méthyl]-1,3-thiazol-4-yl}-6-chromanol

Le protocole expérimental utilisé est analogue à celui décrit pour l'étape 2 de la préparation 8, l'intermédiaire obtenu à l'étape 3 de la présente préparation étant utilisé comme produit de départ à la place de l'intermédiaire obtenu à l'étape 1 de la préparation 8, et le benzène remplaçant le toluène comme solvant. Le produit obtenu est purifié par cristallisation dans un minimum de dichlorométhane pour conduire à un solide blanc avec un rendement de 48%. Point de fusion : 153-155 °C.

### Préparation 11 : chlorhydrate de 3,5-ditert-butyl-4'-{2-[(méthylamino)méthyl]-1,3-thiazol-4-yl}-1,1'-biphényl-4-ol :

### Étape 1 : acide 3',5'-ditert-butyl-4'-hydroxy-1,1'-biphényl-4-carboxylique

5,0 g (1,41 mmol) de 3',5'-ditert-butyl-4'-hydroxy-1,1'-biphényl-4-carboxylate d'éthyle (Chem. Lett. (1998), 9, 931-932) sont dissous dans de l'éthanol (25 ml). La solution est refroidie à 0 °C puis on ajoute goutte à goutte une solution de soude 1*N*. Après agitation une nuit à température ambiante, le milieu réactionnel est porté à reflux pour achever la réaction. Après évaporation des solvants et dilution du résidu avec de l'eau, on acidifie le mélange obtenu avec une solution de HCl 1*N* et on procède à une extraction avec dichlorométhane. La phase organique est lavée avec du chlorure de sodium en solution aqueuse saturée avant d'être ensuite séchée sur sulfate de magnésium, filtrée et concentrée sous vide. Le produit obtenu est purifié par cristallisation dans de l'éther diisopropylique pour conduire à un solide blanc-jaunâtre avec un rendement de 47%. Point de fusion : >240 °C.

### Étape 2 : 3'5'-ditert-butyl-4'-hydroxy-N-méthoxy-N-méthyl-1,1'-biphényl-4-carboxamide

Le protocole expérimental utilisé est identique à celui décrit pour l'étape 1 de la préparation 10, l'acide obtenu à l'étape 1 de la présente préparation remplaçant le Trolox^{®} comme produit de départ. On obtient un solide jaunâtre avec un rendement de 93%. Point de fusion : 175,6-177 °C.

### Étape 3 : 1-(3',5'-ditert-butyl-4'-hydroxy-1,1'-biphényl-4 yl)éthanone

Le protocole expérimental utilisé est identique à celui décrit pour l'étape 2 de la préparation 10, l'intermédiaire obtenu à l'étape 2 de la présente préparation remplaçant l'intermédiaire obtenu à l'étape 1 de la préparation 10. On obtient un solide blanc avec un rendement de 74%. Point de fusion : 144-144,7 °C.

### Étape 4 : 2-bromo-1-(3',5'-ditert-butyl-4'-hydroxy-1,1'-biphényl-4 yl)éthanone

Le protocole expérimental utilisé est identique à celui décrit pour l'étape 3 de la préparation 10, l'intermédiaire obtenu à l'étape 3 de la présente préparation remplaçant l'intermédiaire l'intermédiaire obtenu à l'étape 2 de la préparation 10. On obtient une huile jaune-orange suffisamment pure pour pouvoir être utilisée dans l'étape qui suit (rendement de 100%).

### Étape 5 : [4-(3',5'-ditert-butyl-4'-hydroxy-1,1'-biphényl-4 yl)-1,3-thiazol-2-yl]méthyl(méthyl)carbamate de tert-butyle

Ce composé est préparé selon le protocole expérimental décrit dans l'étape 3 de la préparation 1, en utilisant l'intermédiaire obtenu à l'étape 4 de la présente préparation à la place de la bromo-1-(3,5-ditert-butyl-4-hydroxyphényl)éthanone. Le composé attendu est obtenu sous forme d'une huile incolore avec un rendement de 46%.
MH+ = 509,43.

### Étape 6 : chlorhydrate de 3,5-ditert-butyl-4'-{2-[(méthylamino)méthyl]-1,3-thiazol-4-yl}-1,1'-biphényl-4-ol

0,230 g (0,452 mmol) de l'intermédiaire obtenu à l'étape 5 de la présente préparation sont dissous dans de l'acétate d'éthyle (20 ml). On fait barboter HCl gaz à travers la solution obtenue préalablement refroidie à 0 °C. On laisse ensuite revenir à température ambiante le mélange agité. Le solide formé est filtré et lavé avec de l'acétate d'éthyle puis de l'éther avant d'être séché sous vide. Un solide blanc est obtenu avec un rendement de 85%. Point de fusion : 220-221 °C.

### Préparation 12 : chlorhydrate de 2,6-diméthoxy-4-{2-[(méthylamino)méthyl]-1,3-thiazol-4-yl}phénol

### Étape 1 : acétate de 4-acétyl-2,6-diméthoxyphényle

3,0 g (15,3 mmol) de 3,5-diméthoxy-4-hydroxyacétophénone sont dissous dans du dichlorométhane (30 ml) et 2,53 g (18,3 mmol) de K₂CO₃ sont ajoutés. De la triéthylamine (2,6 ml) est alors ajoutée goutte à goutte. Le milieu réactionnel est refroidi à 0 °C et du chlorure d'acétyle (1,31 ml ; 18,3 mmol) est ajouté. On laisse le mélange agiter 24 heures à température ambiante avant de le verser sur de l'eau glacée. Après extraction avec du dichlorométhane, la phase organique est lavée avec du chlorure de sodium en solution aqueuse saturée avant d'être séchée sur sulfate de magnésium, filtrée et concentrée sous vide. Le produit obtenu est purifié par cristallisation dans de l'éther pour conduire à un solide blanc avec un rendement de 99%. Point de fusion : 145 °C.

### Étape 2 : acétate de 4-(bromoacétyl)-2,6-diméthoxyphényle

L'intermédiaire obtenu à l'étape 1 (0,850 g ; 3,57 mmol) est solubilisé dans de l'acétate d'éthyle puis on ajoute 1,35 g (6,07 mmol) de CuBr₂ préalablement séché. Le mélange est porté à reflux pendant 2 heures et demie avant d'être laissé revenir à température ambiante. Du noir végétal est ajouté et le mélange agité pendant 10 minutes. Après filtration et évaporation à sec, le solide obtenu est repris dans de l'éther diisopropylique. Après filtration, on obtient un solide gris avec un rendement de 75%. Point de fusion : 124,2-126,3 °C.

### Étape 3 : acétate de 4-(2-{[(tert-butoxycarbonyl)(méthyl)amino]méthyl}-1,3-thiazol-4 yl)-2,6-diméthoxyphényle

Ce composé est préparé selon le protocole expérimental décrit pour l'étape 3 de la préparation 1, en utilisant l'intermédiaire obtenu à l'étape 2 de la présente préparation à la place de la bromo-1-(3,5-ditert-butyl-4-hydroxyphényl)éthanone. Le composé attendu est obtenu sous forme d'un solide blanc avec un rendement de 55%. Point de fusion : 135,2-137,4 °C.

### Étape 4 : [4-(4-hydroxy-3,5-diméthoxyphényl)-1,3-thiazol-2-yl]méthyl(méthyl)carbamate de tert-butyle

0,530 g (1,25 mmol) de l'intermédiaire obtenu à l'étape 3 sont dissous dans du méthanol (20 ml). La solution est refroidie à l'aide d'un bain de glace puis une solution de NaOH 1*N* est ajoutée goutte à goutte. On laisse le mélange revenir à température ambiante tout en l'agitant. Après évaporation à sec et dilution à l'eau du résidu, la solution est neutralisée à l'aide d'acide citrique et extraite avec du dichlorométhane. La phase organique est lavée avec du chlorure de sodium en solution aqueuse saturée avant d'être séchée sur sulfate de magnésium, filtrée et concentrée sous vide. Le produit est obtenu sous forme d'une huile jaune avec un rendement de 96%.
MH+ = 381,20.

### Étape 5 : chlorhydrate de 2,6-diméthoxy-4-{2-((méthylamino)méthyl]-1,3-thiazol-4-yl}phénol

Le protocole expérimental utilisé est identique à celui décrit pour l'étape 6 de la Préparation 11, l'intermédiaire obtenu à l'étape 4 de la présente préparation remplaçant l'intermédiaire obtenu à l'étape 5 de la préparation 11. On obtient un solide beige clair avec un rendement de 97%. Point de fusion : 229,8-232,0 °C.

### Préparation 13 : 2,6-ditert-butyl-4-[2-(hydroxyméthyl)-1,3-thiazol-4-yl]phénol

*[il s'agit de l'intermédiaire 6.d₁) de la demande de brevet* EP 432 740]

### Étape 1 : pivalate de [4-(3,5-ditert-butyl-4-hydroxyphényl)-1,3-thiazol-2-yl]méthyle

Ce composé est préparé selon un protocole identique à celui décrit pour l'étape 3 de la préparation 1 en utilisant le 2-(*tert*-butylcarbonyloxy)thioacétamide à la place du 2-{[(1,1-diméthyléthoxy)carbonyl]méthyl}amino-éthanethioamide et le toluène remplaçant le benzène. Le composé attendu est obtenu sous forme d'un solide blanc avec un rendement de 100%. Point de fusion : 114,6-116,0 °C.

### Étape 2 : 2,6-ditert-butyl-4-[2-(hydroxyméthyl)-1,3-thiazol-4-yl]phénol

Le protocole expérimental utilisé est identique à celui décrit pour l'étape 4 de la préparation 12, l'intermédiaire obtenu à l'étape 1 de la présente préparation remplaçant l'intermédiaire obtenu à l'étape 3 de la préparation 12. On obtient un solide blanc avec un rendement de 88%. Point de fusion : 126,4-127,4 °C.

### Préparation 14 : chlorhydrate de 2,6-ditert-butyl-4-{2-[2-(méthylamino)éthyl]-1,3-thiazol-4-yl}phénol :

### Étape 1 : 2-cyanoéthyl(méthyl)carbamate de tert-butyle :

0,1 mol de N-méthyl-β-alaninenitrile est mise en solution dans du dichlorométhane (100 ml) contenant 20,9 ml (0,12 mol) de diisopropyléthylamine. Le mélange est refroidi à 0 °C puis on ajoute par fractions Boc-O-Boc (26,2 g ; 0,12 mol) et on laisse agiter le mélange une nuit à température ambiante. Le milieu réactionnel est alors versé sur de l'eau glacée et extrait au dichlorométhane. La phase organique est lavée successivement avec une solution aqueuse de bicarbonate de sodium à 10% et à l'eau, puis enfin avec une solution de chlorure de sodium saturée. La phase organique est ensuite séchée sur sulfate de magnésium, filtrée et concentrée sous vide. L'huile marron-rouge obtenue est utilisée telle quelle dans l'étape suivante.

### Étape 2 : 3-amino-3-thioxopropyl(méthyl)carbamate de tert-butyle :

43,4 mmol de l'intermédiaire obtenu à l'étape 1 sont dissoutes dans de l'éthanol (40 ml) contenant de la triéthylamine (6,1 ml). On fait ensuite barboter H₂S dans le mélange pendant 3 h avant d'évaporer les solvants à sec. Le produit attendu est obtenu après chromatographie sur une colonne de silice (éluant : 50% d'acetate d'éthyle dans de l'heptane) sous forme d'huile orange clair. La cristillisation de cette huile dans de l'éther diisopropylique donne un solide blanc avec un rendement de 15% Point de fusion : 104 °C.

### Étape 3 : 4-[3,5-bis(1,1-diméthyléthyl)-4-hydroxyphényl]-N-((1,1-diméthyléthoxy)-carbonyl]-N-méthyl-2-thiazoleéthanamine :

L'intermédiaire obtenu à l'étape 2 (2,11 mmol) et de la bromo-1-(3,5-di*tert*-butyl-4-hydroxyphényl)éthanone (6,9 g; 2,11 mmol) sont dissous dans du toluène (75 ml) sous atmosphère d'argon puis le mélange est agité à température ambiante durant 12 heures. Le milieu réactionnel est porté au reflux pendant 4 heures. Après évaporation des solvants, le résidu est dilué avec du dichlorométhane et lavé avec une solution saturée en NaCl. La phase organique est séparée, séchée sur sulfate de magnésium, filtrée et concentrée sous vide. Le produit attendu est cristallisée sous forme d'un solide blanc. Point de fusion : 204 °C.

### Étape 4 : Chlorhydrate de 2,6-ditert-butyl-4-{2-[2-(méthylamino)éthyl]-1,3-thiazol-4 yl}phénol :

1,95 mmol de l'intermédiaire obtenu à l'étape 3 sont dissous dans de l'acétate d'éthyle (20 ml). La solution est refroidie à 0 °C puis on fait barboter HCl gaz pendant 10 minutes. On laisse le mélange revenir à température ambiante tout en le maintenant agité. Après filtration et séchage sous vide, le produit attendu est récupéré sous la forme de cristaux blancs que l'on lave avec de l'éther. Rendement quantitatif. Point de fusion : 206-208 °C.

### Préparation 15 : 2,6-ditert-butyl-4-[2-(méthoxyméthyl)-1,3-thiazol-4-yl]phénol :

Étape 1 : *pivalate de [4-(3,5-ditert-butyl-4-hydroxyphényl)-1,3-thiazol-2 yl]méthyle :*

Ce composé est préparé selon un protocole identique à celui décrit pour l'étape 3 de la préparation 14, en utilisant le 2-(*tert*-butylcarbonyloxy)thioacétamide à la place de l'intermédiaire obtenu à l'étape 2 de la préparation 14 et le toluène remplaçant le benzène. Le composé attendu est obtenu sous forme d'un solide blanc avec un rendement de 100%. Point de fusion : 114,6-116,0 °C.

### Étape 2 : 2,6-ditert-butyl-4-[2-(hydroxyméthyl)-1,3-thiazol-4-yl]phénol :

L'intermédiaire obtenu à l'étape 1 (1,25 mmol) est dissous dans du méthanol (20 ml). La solution est refroidie à l'aide d'un bain de glace puis une solution de NaOH 1*N* est ajoutée goutte à goutte. On laisse le mélange revenir à température ambiante tout en l'agitant. Après évaporation à sec et dilution à l'eau du résidu, la solution est neutralisée à l'aide d'acide citrique et extraite avec du dichlorométhane. La phase organique est lavée avec du chlorure de sodium en solution aqueuse saturée avant d'être séchée sur sulfate de magnésium, filtrée et concentrée sous vide. On obtient un solide blanc avec un rendement de 88%. Point de fusion : 126,4-127,4 °C.

### Étape 3 : 2,6-ditert-butyl-4-(2-(méthoxyméthyl)-1,3-thiazol-4 yl]phénol :

L'intermédiaire obtenu à l'étape 2 (1 équivalent) est méthylé par réaction avec 1,1 équivalent d'iodométhyle en présence de 2 équivalents de triéthylamine, la réaction s'effectuant dans du tétrahydrofuranne. On obtient une poudre crème foncé. Point de fusion : 115,8-117 °C.

### Préparation 16 : 2,6-ditert-butyl-4-[2-(morpholin-4-ylméthyl)-1,3-thiazol-4-yl]phénol :

### Étape 1 : 4-[2-(bromométhyl)-1,3-thiazol-4 yl]-2,6-ditert-butylphénol :

On dissout 1,5 g (4,70 mmol) de l'intermédiaire obtenu à l'étape 2 de la préparation 15, le (2,6-ditert-butyl-4-[2-(hydroxyméthyl)-1,3-thiazol-4-yl]phénol, dans du dichlorométhane (30 ml). Après ajout de CBr₄ (2,02 g ; 6,10 mmol), on refroidit le milieu réactionnel à 0 °C. PPh₃ (1,48 g ; 5,63 mmol) est ajouté par fractions puis le mélange est laissé revenir à température ambiante. Le milieu réactionnel est alors versé sur de l'eau glacée avant d'être extrait au dichlorométhane. La phase organique est lavée à l'eau salée avant d'être séchée sur sulfate de magnésium, filtrée et concentrée sous vide. Le produit attendu est obtenu après chromatographie sur une colonne de silice (éluant : 30% d'acétate d'éthyle dans de l'heptane), pour conduire à une huile marron avec un rendement de 92%. Ce produit est suffisamment pur pour pouvoir être utilisé directement dans l'étape suivante.
MH+ = 382,20.

### Étape 2 : 2,6-ditert-butyl-4-[2-(morpholin-4 ylméthyl)-1,3-thiazol-4-yl]phénol :

1,57 mmol de morpholine et 0,4 ml (2,62 mmol) de triéthylamine sont dissous dans du diméthylformamide (15 ml). 0,400 g (1,05 mmol) de l'intermédiaire obtenu à l'étape 1 dissous dans du diméthylformamide (5 ml) sont ajoutés puis on laisse le mélange agité à température ambiante pendant 18 heures. Le milieu réactionnel est ensuite versé sur de l'eau glacée et extrait avec de l'acétate d'éthyle. La phase organique est lavée à l'eau salée avant d'être séchée sur sulfate de magnésium, filtrée et concentrée sous vide. Le produit attendu est obtenu après chromatographie sur une colonne de silice (éluant : 50% d'acétate d'éthyle dans de l'heptane), pour conduire à une huile orange avec un rendement de 92%. On obtient des cristaux crème clair. Point de fusion : 136,7-137,2 °C.

### Exemple 1 : 2-[4-(4-aminophényl)-1H imidazol-2-yl]éthylcarbamate de butyle (ne fait pas partie de l'invention):

### 1.1) 2-[4-(4-nitrophényl)-1H-imidazol-2-yl]éthylcarbamate de butyle :

Ce composé est préparé selon un protocole identique à celui décrit pour la préparation 9, le bromure de 4-nitrophénacyle remplaçant la 4-phényl-bromoacétophénone dans l'étape 2. Le produit attendu est obtenu sous forme d'une poudre brune avec un rendement de 1 %.
MH+= 333,20.

### 1.2) 2-[4-(4-aminophényl)-1H-imidazol-2-yl]éthylcarbamate de butyle :

L'intermédiaire 1.1 (0,28 g ; 0,84 mmol) est dissous dans 20 ml d'éthanol. 0,02 g de palladium sur carbone (10 %) est ajouté et le mélange est placé sous atmosphère d'hydrogène (2 bars de pression). Le catalyseur est récupéré par filtration puis le solvant est évaporé sous pression réduite. Le produit attendu est purifié par chromatographie sur une colonne de silice (éluant = 8% de méthanol et 0,5% d'ammoniaque dans du dichlorométhane) pour donner une poudre brune avec un rendement de 24%. Point de fusion : 120 °C.

### Exemple 2 : N,2-diméthyl-1-[4-(10H-phénothiazin-2-yl)-1,3-thiazol-2-yl]propan-1-amine (ne fait pas partie de l'invention):

### 2.1) N-[(benzyloxy)carbonyl]-N-méthylvaline :

10,0 g (0,0762 mol) de N-(Me)-(DL)-Valine-OH sont dissous dans un mélange dioxane/eau (90/10 ; 100 ml) et le pH est ajusté à 11 à l'aide d'une solution aqueuse 1*N* d'hydroxide de sodium. Du benzyloxysuccinimide (20,9 g ; 0,0839 mol) dans du dioxane (40 ml) est ajouté goutte à goutte et le mélange agité pendant la nuit at température ambiante. Le milieu réactionnel est ensuite versé dans de l'eau refroidie avec de la glace et acidifié à l'aide d'une solution aqueuse d'acide citrique à 10% avant d'être extrait avec de l'acétate d'éthyle. La phase organique est lavée avec une solution saturée en chlorure de sodium. La phase organique est ensuite séchée sur du sulfate de magnésium, filtrée et concentrée sous vide. Le résidu est purifié sur une colonne de silice (éluant : 5% d'éthanol dans du dichlorométhane) pour donner le composé titre sous forme d'une huile jaune pâle avec un rendement de 64%.
MH+ = 266,10.

### 2.2) N-(méthyl)(CBZ)-(DL)-Valine-NH₂ :

De l'hydroxybenzotriazole (100 g ; 0,653 mol) est suspendu dans du méthanol (500 ml), une solution aqueuse d'hydroxyde d'ammonium à 28% (60 ml) est ajoutée goutte à goutte à température ambiante et la suspension se transforme lentement en solution avant précipitation, l'agitation étant poursuivie pendant environ 5 heures. Le méthanol est évaporé et le solide blanc trituré avec de l'isopropyléther. Le solide est filtré et lavé avec de l'isopropyléther pour donner le complexe HOBT.NH₃ sous forme d'une poudre blanche avec un rendement de 76%.

L'intermédiaire 2.1 (12,9 g ; 0,0486 mol), du HOBT.NH₃ (tel que préparé précédemment; 9,1 g ; 0,0584 mol) et du benzotriazol-1-yloxytris(dimethylamino)phosphonium-hexafluorophosphate (BOP) (21,5 g ; 0,0486 mol) sont dissous dans du DMF (120 ml) sous atmosphère d'argon. Le mélange est refroidi à 0 °C et de la di-isopropyléthylamine (18,7 ml) est ajoutée goutte à goutte. Le mileu réactionnel est laissé revenir à température ambiante avec agitation pendant la nuit. Le milieu réactionnel est ensuite versé dans de l'eau refroidie avec de la glace et une extraction avec de l'acétate d'éthyle est effectuée. La phase organique est lavée avec une solution aqueuse de bicarbonate de sodium à 10% puis une solution saturée en chlorure de sodium. La phase organique est ensuite séchée sur du sulfate de magnésium, filtrée et concentrée sous vide. Le résidu solide est trituré avec de l'éther, le solide est filtré pour donner un solide blanc hygroscopique avec un rendement de 83%, lequel est utilisé directement dans l'étape suivante.
MH+ = 265,20

### 2.3) 1-(aminocarbonothioyl)-2-méthylpropyl(méthyl)carbamate de benzyle :

Ce composé est préparé selon un protocole identique à celui décrit pour l'étape 1.2 de l'exemple 1, l'intermédiaire 2.2 remplaçant l'intermédiaire 1.1. Le produit attendu est obtenu sous forme d'un solide blanc avec un rendement de 36%. Point de fusion : 130 °C.

### 2.4) Méthyl{2-méthyl-1-[4-(10H-phénothiazin-2-yl)-1,3-thiazol-2-yl]propyl}carbamate de benzyle :

Ce composé est préparé selon un protocole identique à celui décrit pour l'étape 1.3 de l'exemple 1, l'intermédiaire 2.3 remplaçant l'intermédiaire 1.2, la 2-chloro-1-[10-(chloroacétyl)-10H-phénothiazin-2-yl)éthanone remplaçant la bromo-1-(3,5-di*tert*-butyl-4-hydroxyphényl)éthanone et le toluène remplaçant le benzène. Le produit attendu est obtained sous forme d'une mousse jaune-orange avec un rendement de 49%.
MH+ = 502,10.

### 2.5) N,2-diméthyl-l-[4-(10H-phénothiazin-2-yl)-1,3-thiazol-2-yl]propan-1-amine :

L'intermédiaire 2.4 (1,2 g ; 0,00238 mol) est dissous dans de l'acide acétique glacial (12 ml). Du HCl concentré (4 ml) est ajouté goutte à goutte et le mélange est ensuite chauffé à 100 °C pendant 2 heures avant d'être évaporé à sec. Le résidu est repris dans du dichlorométhane et lavé avec une solution aqueuse de bicarbonate de sodium à 10% puis avec des solutions saturées en chlorure de sodium jusqu'à ce que la phase aqueuse soit neutre (papier pH). La phase organique est ensuite séchée sur du sulfate de magnésium, filtrée et concentrée sous vide. Le résidu est purifié sur une colonne de silice en phase inversée RP 18 (éluant : soulution aqueuse TFA à 40% (0.1*N*) dans de l'acétonitrile). Les fractions combinées sont évaporées à sec, une solution aqueuse de bicarbonate de sodium à 10% est ajoutée au résidu et le mélange extrait avec du dichlorométhane puis avec une solution saturée en chlorure de sodium. La phase organique est ensuite séchée sur du sulfate de magnésium, filtrée et concentrée sous vide. Le solide est trituré avec de l'isopentane pour donner le composé titre sous forme d'un solide jaune-orange avec un rendement de 14%. Point de fusion : 143,2-144,0 °C.

### Exemple 3 : N,2-diméthyl-1-[4-(10H-phénoxazin-2-yl)-1,3-thiazol-2-yl]propan-1-amine (ne fait pas partie de l'invention):

Le protocole expérimental utilisé est identique à celui décrit pour l'exemple 2, la 2-chloro-1-[10-(chloroacétyl)-10H-phénoxazine-2-yl)éthanone remplaçant la 2-chloro-1-[10-(chloroacétyl)-10H-phénothiazin-2-yl)éthanone pour donner finalement le composé titre sous forme d'une mousse marron. MH+= 352,2.

### Exemple 4 : N,3-diméthyl-1-[4-(10H-phénoxazin-2-yl)-1,3-thiazol-2-yl]butan-1-amine (ne fait pas partie de l'invention):

Le protocole expérimental utilisé est identique à celui décrit pour l'exemple 2, le thioamide 1-(aminocarbonothioyl)-3-méthylbutyl(méthyl)carbamate de benzyle (préparé de la même façon que l'intermédiaire 2.3) remplaçant le 1-(aminocarbonothioyl)-2-méthylpropyl(méthyl)carbamate de benzyle et la 2-chloro-1-[10-(chloroacétyl)-10-H-phénoxazine-2-yl)éthanone remplaçant la 2-chloro-1-[10-(chloroacétyl)-10H-phénothiazin-2-yl)éthanone dans l'étape 2.4 pour donner finalement le composé titre sous forme d'un solide beige. Point de fusion : 143,1-147,0 °C.

### Exemple 5 : N,3-diméthyl-1-[4-(10H-phénothiazin-2-yl)-1,3-thiazol-2-yl]butan-1-amine (ne fait pas partie de l'invention):

Le protocole expérimental utilisé est identique à celui décrit pour l'exemple 2, le thioamide 1-(aminocarbonothioyl)-3-méthylbutyl(méthyl)carbamate de benzyle (préparé de la même façon que l'intermédiaire 2.3) remplaçant le 1-(aminocarbonothioyl)-2-méthylpropyl(méthyl)carbamate de benzyle dans l'étape 2.4, pour donner finalement le composé titre sous forme de cristaux jaunes. Point de fusion : 145,7-148,1 °C.

### Exemple 6 : sel chlorhydrate du 2,6-di-tert-butyl-4-{2-[3-méthyl-1-(méthylamino)butyl]-1,3-thiazol-4-yl}phénol :

Le protocole expérimental utilisé est identique à celui décrit pour l'exemple 2, le thioamide 1-(aminocarbonothioyl)-3-méthylbutyl(méthyl)carbamate de benzyle (préparé de la même façon que l'intermédiaire 2.3) remplaçant le 1-(aminocarbonothioyl)-2-méthylpropyl(méthyl)carbamate de benzyle et la 2-bromo-1-(3,5-ditert-butyl-4-hydroxyphényl)éthanone remplaçant la 2-chloro-1-[10-(chloroacétyl)-10H-phénothiazin-2-yl)éthanone dans l'étape 2.4 pendant laquelle l'élimination du groupe protecteur CBZ se produit *in situ.* Le composé base libre résultant est purifié par chromatographie en phase normale sur une colonne de gel de silice (éluant : 30% d'acétate d'éthyle dans l'heptane). Après traitement de la base libre à l'aide de HCl 1*N* dans de l'éther, on obtient le composé titre sous forme d'un solide blanc-crémeux avec un rendement global de 13%. Point de fusion : 148,1-149,0 °C.

### Exemple 7 : sel chlorhydrate de la [4-(3,5-di-tert-butylphényl)-1,3-thiazol-2-yl]méthylamine (ne fait pas partie de l'invention):

Le protocole expérimental utilisé est identique à celui décrit pour l'exemple 2, le thioamide 2-amino-2-thioxoéthylcarbamate de benzyle (préparé de la même façon que l'intermédiaire 2.3) remplaçant l'intermédiaire 2.3 et la 2-bromo-1-(3,5-di*tert*-butyl-phényl)éthanone remplaçant la 2-chloro-1-[10-(chloroacétyl)-10H-phénothiazin-2-yl)éthanone dans l'étape 2.4. La déprotection du groupe protecteur CBZ est effectuée de la même façon que dans l'étape 2.5. Le composé base libre formé est purifié par chromatographie en phase normale sur une colonne de gel de silice (éluant : 10% d'acétate d'éthyle dans de l'heptane). Après traitement de la base libre à l'aide de HCl 1*N* dans de l'éther, on obtient le composé titre sous forme d'un solide blanc crémeux. Point de fusion : 207,0-209,6 °C.

### Exemple 8 : sel chlorhydrate du 2,6-di-tert-butyl-4-{2-[(1S)-1-(méthylamino)éthyl]-1,3-thiazol-4-yl}phénol :

Le protocole expérimental utilisé est identique à celui décrit pour l'exemple 2, le thioamide (*1S*)-2-amino-1-méthyl-2-thioxoéthyl(méthyl)carbamate de benzyle remplaçant l'intermédiaire 2.3 et la 2-bromo-1-(3,5-di*tert*-butyl-4-hydroxyphényl)éthanone remplaçant la 2-chloro-1-[10-(chloroacétyl)-10H-phénothiazin-2-yl)éthanone. La déprotection du groupe protecteur CBZ est effectuée de la même façon que dans l'étape 2.5. Le composé base libre formé est purifié par chromatographie en phase normale sur une colonne de gel de silice (éluant : 3% d'éthanol dans du dichlorométhane). Après traitement de la base libre à l'aide de HCl 1*N* dans de l'éther, on obtient le composé titre sous forme d'un solide blanc cristallin. Point de fusion : 240,6-242,0 °C.

### Exemple 9 : sel chlorhydrate du 2,6-di-tert-butyl-4-{2-[(1R)-1-(méthylamino)éthyl]-1,3-thiazol-4-yl}phénol :

Le protocole expérimental utilisé est identique à celui décrit pour l'exemple 8, le thioamide (*1R*)-2-amino-1-méthyl-2-thioxoéthyl(méthyl)carbamate de benzyle remplaçant le thioamide (*1S*)-2-amino-1-méthyl-2-thioxoéthyl(méthyl)carbamate de benzyle, pour finalement donner après formation du sel le composé titre sous forme d'un solide blanc cristallin. Point de fusion : 242,8-243,6 °C.

### Exemple 10 : sel chlorhydrate de la N-{[4-(3,5-di-tert-butylphényl)-1,3-thiazol-2-yl]méthyl}-N-méthylamine (ne fait pas partie de l'invention):

Le protocole expérimental utilisé est identique à celui décrit pour la préparation 1, la 2-bromo-1-(3,5-di*tert*-butyl-phényl)éthanone remplaçant la 2-bromo-1-(3,5-di*tert*-butyl-4-hydroxyphényl)éthanone dans l'étape 3. L'élimination du groupe protecteur N-(Boc) et la formation du sel sont effectuées en une étape à l'aide de HCl gaz selon un protocole similaire à celui décrit pour la préparation 7, étape 2 pour donner le composé titre sous forme d'un solide blanc crémeux. Point de fusion : 212,2-213,9 °C.

### Exemple 11 : sel chlorhydrate de la N-méthyl-N-{[4-(3,4,5-triméthoxyphényl)-1,3-thiazol-2-yl]méthyl}amine (ne fait pas partie de l'invention):

### 11.1) 2-bromo-1-(3,4,5-triméthoxyphényl)éthanone :

Le protocole expérimental utilisé est identique à celui décrit pour la préparation 12, étape 2, la 3,4,5-triméthoxy-acétophénone disponible commercialement remplaçant l'intermédiaire obtenu à l'étape 1 de la préparation 12. L'intermédiaire 11.1 est obtenu après chromatographie sur une colonne de silice (éluant : 50% d'acétate d'éthyle dans de l'heptane) sous forme d'un solide jaune avec un rendement de 66%.
MH+= 289,01.

### 11.2) Sel chlorhydrate de la N-méthyl-N-{(4-(3,4,5-triméthoxyphényl)-1,3-thiazol-2-yl]méthyl}amine :

Ce composé est obtenu en utilisant le même protocole que celui décrit pour la préparation 1, l'intermédiaire 11.1 remplaçant la 2-bromo-1-(3,5-di*tert*-butyl-4-hydroxyphényl)éthanone dans l'étape 4. L'élimination du groupe protecteur Boc et la formation du sel sont effectuées en une étape à l'aide de HCl gaz selon un protocole similaire à celui décrit pour la préparation 7, étape 2 pour donner le composé titre sous forme d'un solide jaune cristallin. Point de fusion : 199,4-200,6 °C.

### Exemple 12 : sel chlorhydrate du N-{[4-(3,5-di-tert-butyl-4-hydroxyphényl)-1,3-thiazol-2-yl]méthyl}glycinate d'éthyle (ne fait pas partie de l'invention):

Le protocole expérimental utilisé est identique à celui décrit pour la préparation 2, le bromoacétate d'éthyle remplaçant le chloropropargyle et le composé de la préparation 4 remplaçant le composé de la préparation 1. Après traitement de la base libre à l'aide de HCl 1*N* dans de l'éther, on obtient le composé titre sous forme d'un solide blanc cristallin avec un rendement global de 69%. Point de fusion : 164,0-167,0 °C.

### Exemple 13 : sel chlorhydrate de la N-{[4-(3,5-di-tert-butyl-4-hydroxyphényl)-1,3-thiazol-2-yl]méthyl}glycine (ne fait pas partie de l'invention):

### 13.1) N-(tert-butoxycarbonyl)-N-{[4-(3,5-di-tert-butyl-4-hydroxyphényl)-1,3-thiazol-2-yl]méthyl}glycinate d'éthyle :

Le protocole expérimental utilisé est identique à celui décrit pour la préparation 14, étape 1, le composé de l'exemple 12 remplaçant le N-méthyl-β-alaninenitrile, la triéthylamine étant utilisée à la place de la diisopropylethylamine et une quantité catalytique de diméthylaminopyridine (DMAP) étant ajoutée pour effectuer la réaction. Le composé titre est obtenu sous forme d'une huile verte qui est utilisée directement dans l'étape suivante.
MH+ = 505,30.

### 13.2) N-(tert-butoxycarbonyl)-N-{[4-(3,5-di-tert-butyl-4-hydroxyphényl)-1,3-thiazol-2-yl]méthyl}glycine :

1.0 g (1,98 mmol) de l'intermédiaire 13.1 est dissous dans du THF (20 ml). Une solution d'hydroxide de lithium (1*N* dans l'eau) est ajoutée goutte à goutte et le milieu réactionnel est laissé agité à température ambiante pendant 6 heures. Le milieu réactionnel est ensuite versé dans de l'eau avant d'être extrait avec du diéthyléther. La phase aqueuse est acidifiée avec HCl aqueux (1*N*) et extrait avec du diéthyléther. La phase organique est lavée avec une solution saturée en chlorure de sodium, puis séchée sur du sulfate de magnésium, filtrée et concentrée sous vide avant d'être utilisée directement dans l'étape suivante.
MH+ = 477,20.

### 13.3) Sel chlorhydrate de la N-{[4-(3,5-di-tert-butyl-4-hydroxyphényl)-1,3-thiazol-2-yl]méthyl}glycine :

Le protocole expérimental utilisé est identique à celui décrit pour la préparation 7, étape 2, l'intermédiaire 13.2 remplaçant l'intermédiaire obtenu à l'étape 1 de la préparation 7. Le composé titre est obtenu sous forme d'un solide blanc cristallin avec un rendement de 27%.
MH+ = 377,2.

### Exemple 14 : sel chlorhydrate du 2,6-di-tert-butyl-4-{2-[(4-méthoxypipéridin-1-yl)méthyl]-1,3-thiazol-4-yl}phénol (ne fait pas partie de l'invention):

Le protocole expérimental utilisé est identique à celui décrit pour la préparation 16, étape 2, la 4-méthoxy-pipéridine remplaçant la morpholine. Le composé titre est obtenu sous forme d'un solide blanc cristallin. Point de fusion : 198,0-201,0 °C.

### Exemple 15 : sel chlorhydrate de la N-méthyl-N-{(1S)-2-méthyl-1-[4-(10H-phénothiazin-2-yl)-1,3-thiazol-2-yl]propyl}amine (ne fait pas partie de l'invention):

Ce composé est préparé de façon analogue au composé de l'exemple 2, en utilisant toutefois un réactif de départ optiquement pur, à savoir N-(Me)-(L)-Valine-OH au lieu de N-(Me)-(DL)-Valine-OH). Point de fusion : 270,0-270,8 °C.

### Exemple 16 : sel chlorhydrate de la N,2-diméthyl-1-[4-(10-méthyl-10H-phénothiazin-2-yl)-1,3-thiazol-2-yl]propan-1-amine (ne fait pas partie de l'invention):

### 16.1) {2-méthyl-1-[4-(10-méthyl-10H-phénothiazin-2-yl)-1,3-thiazol-2-yl]propyl}carbamate de benzylméthyle :

L'intermédiaire 2.4 est méthylé selon la procédure suivante : 0,200 g (0,410 mmol) de l'intermédiaire 2.4 est dissous dans du dioxane (10 ml). De l'hydrure de sodium (0,024 g ; 0,598 mmol) est ajouté par petites portions et le mélange est laissé agité pendant 30 minutes. De l'iodométhane (0,04 ml) est ajouté goutte à goutte et chauffé à 45 °C pendant 18 heures. De l'éthanol (10 ml) est ajouté goutte à goutte et le milieu réactionnel est ensuite versé dans de l'eau avant d'être extrait avec de l'acétate d'éthyle. La phase organique est lavée avec une solution saturée en chlorure de sodium, puis séchée sur du sulfate de magnésium, filtrée et concentrée sous vide. L'intermédiaire 16.1 est obtenu après chromatographie sur une colonne de silice (éluant : 15% d'acétate d'éthyle dans de l'heptane) sous forme d'un solide jaune gommeux avec un rendement de 42%.
MH+ = 516,10.

### 16.2) Sel chlorhydrate de la N,2-diméthyl-1-(4-(10-méthyl-10H-phénothiàzin-2-yl)-1,3-thiazol-2-yl]propan-1-amine :

L'intermédiaire 16.1 est dissous dans un mélange acide acétique glacial/eau/méthanol (30 ml). Une quantité catalytique de Pd/C est ajoutée et le milieu réactionnel est hydrogéné à température ambiante sous une pression de 5 bars pendant 12 heures. Le catalyseur épuisé est éliminé par filtration et le filtrat est évaporé à sec et une distillation azéotropique avec du toluène est effectuée plusieurs fois. Le sel chlorhydrate, un solide gris, est obtenu à l'aide d'une solution de HCl 1*N* dans de l'éther avec un rendement global de 45%.
MH+ = 3 82,10.

### Exemple 17 : sel chlorhydrate de la N-méthyl-N-{(1S)-2-méthyl-1-[4-(10H-phénoxazin-2-yl)-1,3-thiazol-2-yl]propyl}amine (ne fait pas partie de l'invention):

Ce composé est préparé de façon analogue au composé de l'exemple 3, en utilisant toutefois un réactif de départ optiquement pur, à savoir N-(Me)-(CBZ)-(L)-Valine-OH au lieu de N-(Me)-(DL)-Valine-OH. On obtient une poudre grise.
MH+ = 352,2.

### Exemple 18 : sel chlorhydrate du 4-{2-[(1R)-1-aminoéthyl]-1,3-thiazol-4-yl}-2,6-di-tert-butylphénol :

Le protocole expérimental utilisé est identique à celui décrit pour le composé de l'exemple 2, le thioamide (*1R*)-2-amino-1-méthyl-2-thioxoéthylcarbamate de benzyle (préparé de façon similaire à l'intermédiaire 2.3) remplaçant le thioamide 1-(aminocarbonothioyl)-2-méthylpropyl(méthyl)carbamate de benzyle et la 2-bromo-1-(3,5-di*tert*-butyl-4-hydroxyphényl)éthanone remplaçant la 2-chloro-1-[10-(chloroacétyl)-10H-phénothiazin-2-yl)éthanone dans la quatrième étape. Le sel chlorhydrate du titre est ensuite obtenu sous forme d'un solide blanc à l'aide de HCl 1*N* dans de l'éther. Point de fusion : 211,8-215,2 °C.

### Exemple 19 : sel chlorhydrate du 4-{2-[(1S)-1-aminoéthyl]-1,3-thiazol-4-yl}-2,6-di-tert-butylphénol :

Le protocole expérimental utilisé est identique à celui décrit pour l'exemple 2, le thioamide (*1S*)-2-amino-1-méthyl-2-thioxoéthylcarbamate de benzyle (préparé de façon similaire à l'intermédiaire 2.3) remplaçant le thioamide 1-(aminocarbonothioyl)-2-méthylpropyl(méthyl)carbamate de benzyle et la 2-bromo-1-(3,5-di*tert*-butyl-4-hydroxyphényl)éthanone remplaçant la 2-chloro-1-[10-(chloroacétyl)-10H-phénothiazin-2-yl)éthanone dans la quatrième étape. Le sel chlorhydrate du titre est ensuite obtenu sous forme d'un solide blanc à l'aide de HCl 1*N* dans de l'éther. Point de fusion : 191,0-195,0 °C.

### Exemple 20: sel chlorhydrate du 4-[2-(1-aminocyclopropyl)-1,3-thiazol-4-yl]-2,6-di-tert-butylphénol :

### 20.1) [1-(aminocarbonyl)cyclopropyl]carbamate de tert-butyle :

Le protocole expérimental utilisé est identique à celui décrit pour l'étape 2 de l'exemple 2, l'acide 1-[(tert-butoxycarbonyl)amino]cyclopropanecarboxylique remplaçant l'intermédiaire 2.1. Le composé titre est obtenu sous forme d'une huile incolore utilisée directement dans l'étape suivante.

### 20.2) [1-(aminocarbonothioyl)cyclopropyl]carbamate de tert-butyle :

Ce composé est préparé selon un protocole identique à celui décrit pour l'étape 2 de la préparation 1, l'intermédiaire 20.1 remplaçant l'intermédiaire obtenu à l'étape 1 de la préparation 1. Le produit attendu est obtained sous forme d'une huile jaune utilisée directement dans l'étape suivante.

### 20.3) Sel chlorhydrate du 4-[2-(1-aminocyclopropyl)-1,3-thiazol-4-yl]-2,6-di-tert-butylphénol:

Ce composé est préparé selon un protocole identique à celui décrit pour l'étape 3 de la préparation 1, l'intermédiaire 20.2 remplaçant l'intermédiaire obtenu à l'étape 2 de la préparation 1 et le toluène remplaçant le benzène. La déprotection du groupe protecteur N-(Boc) se produit *in-situ.* Le sel chlorhydrate est ensuite obtenu sous forme d'un solide blanc crémeux à l'aide de HCl 1*N* dans de l'éther. Point de fusion : 200,6-202,2 °C.

### Exemple 21 : sel chlorhydrate du 4-{2-[(méthylamino)méthyl]-1,3-thiazol-4-yl}benzène-1,2-diol (ne fait pas partie de l'invention):

Le protocole expérimental utilisé est identique à celui décrit pour la préparation 1, la 2-chloro-3',4'-dihydroxyacétophénone remplaçant la 2-bromo-1-(3,5-di*tert*-butyl-4-hydroxyphényl)éthanone. Le composé titre est obtenu sous forme d'un solide blanc crémeux.
MH+ = 237,0.

### Exemple 22 : sel chlorhydrate de la N-méthyl-N-{(1R)-2-méthyl-1-[4-(10H-phénothiazin-2-yl)-1,3-thiazol-2-yl]propyl}amine (ne fait pas partie de l'invention):

Ce composé est préparé de façon analogue au composé de l'exemple 2, en utilisant toutefois un réactif de départ optiquement pur, à savoir N-(Me)-(CBZ)-(D)-Valine-OH au lieu de N-(Me)-(DL)-Valine-OH. On obtient le composé titre sous forme d'une poudre légèrement verte. Point de fusion : 265,6-268,9 °C.

### Exemple 23 : sel chlorhydrate de la (1R)-2-méthyl-1-[4-(10H-phénothiazin-2-yl)-1,3-thiazol-2-yl]propan-1-amine (ne fait pas partie de l'invention):

### 23.1) (1R)-1-[4-(10-acétyl-10H-phénothiazin-2-yl)-1,3-thiazol-2-yl]-2-méthylpropylcarbamate de tert-butyle :

Le protocole expérimental utilisé est identique à celui décrit pour la préparation 1, la 1-(10-acétyl-10H-phénothiazin-2-yl)-2-bromoéthanone *(*Arzneimittel Forschung (1962), 12, 48-52) remplaçant la 2-bromo-1-(3,5-di*tert*-butyl-4-hydroxyphényl)éthanone et le thioamide (*1R*)-1-(aminocarbonothioyl)-2-méthylpropylcarbamate de *tert*-butyle (préparé de façon similaire à l'intermédiaire obtenu à l'étape 2 de la préparation 1) remplaçant l'intermédiaire obtenu à l'étape 2 de la préparation 1. L'élimination du groupe protecteur N-(Boc) et la formation du sel sont effectuées en une étape en utilisant HCl gaz selon une procédure similaire à celle décrite pour l'étape 2 de la préparation 7. Le composé titre est obtenu sous forme d'un solide gris.
MH+ = 396,1.

### 23.2) Sel chlorhydrate de la (1R)-2-méthyl-1-[4-(10H-phénothiazin-2-yl)-1,3-thiazol-2-yl]propan-1-aminé :

L'intermédiaire 23.1 est dissous dans HCl 2*N* et chauffé à reflux pendant 18 heures. The solution est extracted avec acétate d'éthyle et la phase aqueuse est rendue basique à l'aide d'une solution aqueuse de bicarbonate de sodium (10%) et extraite avec de l'acétate d'éthyle. La phase organique est lavée avec une solution aqueuse de bicarbonate de sodium à 10% puis avec une solution saturée en chlorure de sodium. La phase organique est séchée sur du sulfate de magnésium, filtrée et concentrée sous vide. Le résidu est purifié sur une colonne de silice (éluant : 5% d'éthanol dans du dichlorométhane), donnant la base libre sous forme d'une huile brun pâle. Le sel chlorhydrate est obtenu sous forme d'une poudre verte en utilisant HCl 1*N* dans de l'éther.
MH+ = 354,2.

### Exemple 24 : sel chlorhydrate de la N-méthyl-N-{(1R)-2-méthyl-1-[4-(10H-phénoxazin-2-yl)1,3-thiazol-2-yi]propyl}amine (ne fait pas partie de l'invention):

Ce composé est préparé de façon analogue aux étapes 2.2 à 2.5 de l'exemple 2, en utilisant toutefois un réactif de départ optiquement pur, à savoir N-(Me)-(CBZ)-(D)-Valine-OH au lieu de l'intermédiaire 2.1. Le sel chlorhydrate du titre est obtenu sous forme d'une poudre jaune.
MH+ = 352,2.

### Exemple 25 : sel chlorhydrate du N²-{[4-(3,5-di-tert-butyl-4-hydroxyphényl)-1,3-thiazol-2-yl]méthyl}glycinamide (ne fait pas partie de l'invention):

Le protocole expérimental utilisé est identique à celui décrit pour la préparation 2, le 2-bromoacétamide remplaçant le chloropropargyle et le composé de la préparation 4 remplaçant le composé de la préparation 1. Le sel chlorhydrate est ensuite obtenu sous forme d'une poudre blanche à l'aide de HCl 1*N* dans de l'éther.
MH+ = 376,2.

### Exemple 26 : sel chlorhydrate du N-{[4-(3,5-di-tert-butyl-4-hydroxyphényl)-1,3-thiazol-2-yl]méthyl}-N-(2-éthoxy-2-oxoéthyl)glycinate d'éthyle (ne fait pas partie de l'invention):

Le protocole expérimental utilisé est identique à celui décrit pour la préparation 2, avec toutefois un excès de bromoacétate d'éthyle remplaçant le chloropropargyle et le composé de la préparation 4 remplaçant le composé de la préparation 1. Le sel chlorhydrate est ensuite obtenu sous forme d'une mousse blanche à l'aide de HCl 1*N* dans de l'éther.
MH+ = 491,2.

### Exemple 27 : sel chlorhydrate du 4-(3,5-di-tert-butyl-4-méthoxyphényl)-2-(méthoxyméthyl)-1,3-thiazole (ne fait pas partie de l'invention):

Le protocole expérimental utilisé est identique à celui décrit pour l'étape 16.1 de l'exemple 16, l'intermédiaire obtenu à l'étape 1 de la préparation 13 remplaçant l'intermédiaire 2.4 et le THF remplaçant le dioxane. Le composé titre est obtenu sous forme d'un solide blanc avec un rendement de 38%. Point de fusion : 94,0-94,8 °C.

### Exemple 28 : sel chlorhydrate du 4-[2-(1-aminocyclopentyl)-1,3-thiazol-4-yl]-2,6-di-tert-butylphénol :

### 28.1) [1-(aminocarbonyl)cyclopentyl]carbamate de tert-butyle :

Le protocole expérimental utilisé est identique à celui décrit pour l'étape 2 de l'exemple 2, l'acide 1-[(tert-butoxycarbonyl)amino]cyclopentanecarboxylique remplaçant l'intermédiaire 2.1. Le composé titre est obtenu sous forme d'un solide blanc floconneux qui est utilisé directement dans l'étape suivante.

### 28.2) [1-(aminocarbonothioyl)cyclopentyl]carbamate de tert-butyle :

Ce composé est préparé selon un protocole identique à celui décrit pour l'étape 2 de la préparation 1, l'intermédiaire 28.1 remplaçant l'intermédiaire obtenu à l'étape 1 de la préparation 1. Le produit attendu est obtenu sous forme d'un solide blanc floconneux qui est utilisé directement dans l'étape suivante.
MH+ = 245.2.

### 28.3) {1-[4-(3,5-di-tert-butyl-4-hydroxyphenyl)-1,3-thiazol-2-yl]cyclopentyl}carbamate de tert-butyle :

Ce composé est préparé selon un protocole identique à celui décrit pour l'étape 3 de la préparation 1, l'intermédiaire 28.2 remplaçant l'intermédiaire obtenu à l'étape 2 de la préparation 1 et le toluène remplaçant le benzène. Le produit attendu est obtenu sous forme d'une huile incolore.
MH+ = 473,4.

### 28.4) Sel chlorhydrate du 4-[2-(1-aminocyclopentyl)-1,3-thiazol-4-yl]-2, 6-di-tert-butylphenol :

L'élimination du groupe protecteur N-(Boc) de l'intermédiaire 28.3 et la formation du sel sont effectuées en une seule étape à l'aide de HCl gaz selon un protocole similaire à celui décrit pour l'étape 2 de la préparation 7. Le composé titre est obtenu sous forme d'un solide cristallin blanc. Point de fusion : 288,8-290,7 °C.

### Etude pharmacologique des produits de l'invention

Etude des effets sur la liaison d'un ligand spécifique de la MAO-B, le [³H]Ro 19-6327

L'activité inhibitrice des produits de l'invention est déterminée par la mesure de leurs effets sur la liaison d'un ligand spécifique de la MAO-B, le [³H]Ro 19-6327.

### a) Préparation mitochondriale de cortex de rats

La préparation mitochondriale de cortex de rats est réalisée selon la méthode décrite dans Cesura A M, Galva M D, Imhof R et Da Prada M, J. Neurochem. 48 (1987), 170-176. Les rats sont décapités et leurs cortex prélevés, homogénéisés dans 9 volumes d'un tampon sucrose 0,32 M tamponné à pH 7,4 avec 5 mM d'HEPES, puis centrifugés à 800 g pendant 20 minutes. Les surnageants sont récupérés et les culots lavés 2 fois avec le tampon sucrose 0,32 M comme précédemment. Les surnageants récoltés sont centrifugés à 10000 g pendant 20 minutes. Les culots obtenus sont mis en suspension dans un tampon Tris (50 mM Tris, 130 mM NaCl, 5 mM KCl, 0,5 mM EGTA, 1 mM MgCl₂, pH 7,4) et centrifugés à 10000 g pendant 20 minutes. Cette étape est répétée 2 fois, et le culot final, correspondant à la fraction mitochondriale, est conservé à -80 °C dans le tampon Tris. Le contenu protéique de la préparation est déterminé par la méthode de Lowry.

### b) Liaison du [³H]Ro 19-6327

Dans un tube Eppendorf, 100 µl de la préparation mitochondriale (2 mg protéine/ml) sont incubés pendant 1 heure à 37 °C en présence de 100 µl de [³H] Ro 19-6327 (33 nM, concentration finale) et 100 µl de tampon Tris contenant ou non les inhibiteurs. La réaction est arrêtée par l'addition de 1 ml de tampon Tris froid dans chaque tube, puis les échantillons sont centrifugés 2 minutes à 12000 g. Les surnageants sont aspirés et les culots lavés avec 1 ml de tampon Tris. Les culots sont ensuite solubilisés dans 200 (µl de sodium dodécyl sulfate (20% poids/volume) pendant 2 heures à 70 °C. La radioactivité est déterminée par comptage des échantillons en scintillation liquide.

### c) Résultats

Le composé de l'exemple 9 décrit ci-dessus présente une CI₅₀ inférieure à 10 µM. En outre, les composés des exemples 8, 18 et 19 décrits ci-dessus présentent une CI₅₀ inférieure à 20 µM.

### Etude des effets sur la peroxydation lipidique du cortex cérébral de rat

L'activité inhibitrice des produits de l'invention est déterminée par la mesure de leurs effets sur le degré de peroxydation lipidique, déterminée par la concentration en malondialdéhyde (MDA). Le MDA produit par la peroxydation des acides gras insaturés est un bon indice de la peroxydation lipidique (H Esterbauer et KH Cheeseman, Meth. Enzymol. (1990) 186 : 407-421). Des rats mâles Sprague Dawley de 200 à 250 g (Charles River) ont été sacrifiés par décapitation. Le cortex cérébral est prélevé, puis homogénéisé au potter de Thomas dans du tampon Tris-HCl 20 mM, pH = 7,4. L'homogénat est centrifugé deux fois à 50000 g pendant 10 minutes à 4 °C. Le culot est conservé à -80 °C. Le jour de l'expérience, le culot est remis en suspension à la concentration de 1 g / 15 ml et centrifugé à 515 g pendant 10 minutes à 4 °C. Le surnageant est utilisé immédiatement pour la détermination de la peroxidation lipidique. L'homogénat de cortex cérébral de rat (500 µl) est incubé à 37 °C pendant 15 minutes en présence des composés à tester ou du solvant (10 µl). La réaction de peroxydation lipidique est initiée par l'ajout de 50 µl de FeCl₂ à 1 mM, d'EDTA à 1 mM et d'acide ascorbique à 4 mM. Après 30 minutes d'incubation à 37 °C la réaction est arrêtée par l'ajout de 50 µl d'une solution de di tertio butyl toluène hydroxylé (BHT, 0,2 %). Le MDA est quantifié à l'aide d'un test colorimétrique, en faisant réagir un réactif chromogène (R) le N-méthyl-2-phénylindole (650 µl) avec 200 µl de l'homogénat pendant 1 heure à 45 °C. La condensation d'une molécule de MDA avec deux molécules de réactif R produit un chromophore stable dont la longueur d'onde d'absorbance maximale est égale à 586 nm. (Caldwell et coll. European J. Pharmacol. (1995) 285, 203-206). Les composés des exemples 2 à 6, 8, 9 et 12 à 26 décrits ci-dessus présentent une CI₅₀ inférieure à 10 µM.

### Test de liaison sur les canaux sodiques de cortex cérébraux de rat

Le test consiste à mesurer l'interaction des composés vis-à-vis de la liaison de la batrachotoxine tritiée sur les canaux sodiques dépendants du voltage selon le protocole décrit par Brown (J. Neurosci. (1986), 6, 2064-2070).

### Préparation des homogénats de cortex cérébraux de rat

Les cortex cérébraux de rat Sprague-Dawley de 230-250 g (Charles River, France) sont prélevés, pesés et homogénéisés à l'aide d'un broyeur Potter muni d'un piston en téflon (10 allers/retours) dans 10 volumes de tampon d'isolement dont la composition est la suivante (sucrose 0,32 M ; K₂HPO₄ 5 mM ; pH 7,4). L'homogénat subit une première centrifugation à 1000 g pendant 10 minutes. Le surnageant est prélevé et centrifugé à 20000 g pendant 15 minutes. Le culot est repris dans le tampon d'isolement et centrifugé à 20000 g pendant 15 minutes. Le culot obtenu est remis en suspension dans du tampon d'incubation (HEPES 50 mM ; KCl 5,4 mM ; MgSO₄ 0,8 mM ; glucose 5,5 mM ; chlorure de choline 130 mM pH 7,4) puis aliquoté et conservé à -80 °C jusqu'au jour du dosage. La concentration finale en protéines est comprise entre 4 et 8 mg/ml. Le dosage de protéines se fait par un kit commercialisé par BioRad (France).

### Mesure de la liaison de la batrachotoxine tritiée

La réaction de liaison se fait en incubant pendant 1 h 30 à 25 °C 100 (µl d'homogénat de cortex de rat contenant 75 µg de protéines avec 100 µl de [³H] batrachotoxine-A 20-alpha benzoate (37,5 Ci/mmol, NEN) à 5 nM (concentration finale), 200 µl de tétrodotoxine à 1 µM (concentration finale) et de venin de scorpion à 40 µg/ml (concentration finale) et 100 µl de tampon d'incubation seul ou en présence des produits à tester aux différentes concentrations. La liaison non spécifique est déterminée en présence de 300 µM de veratridine et la valeur de cette liaison non spécifique est soustraite à toutes les autres valeurs. Les échantillons sont ensuite filtrés à l'aide d'un Brandel (Gaithersburg, Maryland, USA) en utilisant des plaques Unifilter GF/C préincubées avec 0,1 % de polyéthylène imine (20 µl/puits) et rincées 2 fois avec 2 ml de tampon de filtration (HEPES 5 mM ; CaCl₂ 1,8 mM ; MgSO₄ 0,8 mM ; chlorure de choline 130 mM ; BSA 0,01 % ; pH 7,4). Après avoir ajouté 20 µl de Microscint 0 ^{®}, la radioactivité est comptée à l'aide d'un compteur à scintillation liquide (Topcount, Packard). La mesure est réalisée en duplicat. Les résultats sont exprimés en % de la liaison spécifique de la batrachotoxine tritiée par rapport au témoin.

### Résultats

Les composés des exemples 1, 8 à 10 et 25 décrits ci-dessus présentent tous une CI₅₀ inférieure ou égale à 1 µM.

## Revendications

1. Composé choisi parmi les composés suivants :
- 2,6-di-*tert*-butyl-4-{2-[3-méthyl-1-(méthylamino)butyl]-1,3-thiazol-4-yl}phénol ;
- 2,6-di-*tert*-butyl-4-{2-[(1S)-1-(méthylamino)éthyl]-1,3-thiazol-4-yl}phénol ;
- 2,6-di-*tert*-butyl-4-{2-[(*1R*)-I-(méthylamino)éthyl]-1,3-thiazol-4-yl}phénol ;
- 4-{2-[(*1R*)-1-aminoéthyl]-1,3-thiazol-4-yl}-2,6-di-*tert*-butylphénol ;
- 4-{2-[(*1S*)-1-aminoéthyl]-1,3-thiazol-4-yl}-2,6-di-*tert*-butylphénol ;
- 4-[2-(1-aminocyclopropyl)-1,3-thiazol-4-yl]-2,6-di-*tert*-butylphénol ;
- 4-[2-(1-aminocyclopentyl)-1,3-thiazol-4-yl]-2,6-di-tert-butylphénol ;
ou sel d'un de ces derniers.

2. Composé ou sel selon la revendication 1, **caractérisé en ce qu'**il s'agit du 4-[2-(1-aminocyclopentyl)-1,3-thiazol-4-yl]-2,6-di-tert-butylphénol ou d'un de ses sels.

3. Composé choisi parmi les composés suivants :
- 2,6-di-*tert*-butyl-4-{2-[3-méthyl-1-(méthylamino)butyl]-1,3-thiazol-4-yl}phénol ;
- 2,6-di-*tert*-butyl-4-{2-[(1S)-1-(méthylamino)éthyl]-1,3-thiazol-4-yl}phénol ;
- 2,6-di-*tert*-butyl-4-{2-[(*1R*)-1-(méthylamino)éthyl]-1,3-thiazol-4-yl}phénol ;
- 4-{2-[(*1R*)-1-aminoéthyl]-1,3-thiazol-4-yl}-2,6-di-*tert*-butylphénol ;
- 4-{2-[(*1S*)-1-aminoéthyl]-1,3-thiazol-4-yl}-2,6-di-*tert*-butylphénol ;
- 4-[2-(1-aminocyclopropyl)-1,3-thiazol-4-yl]-2,6-di-*tert*-butylphénol ;
- 4-[2-(1-aminocyclopentyl)-1,3-thiazol-4-yl]-2,6-di-tert-butylphénol ;
ou un sel pharmaceutiquement acceptable d'un de ces derniers, pour son utilisation en tant que médicament.

4. Composition pharmaceutique contenant, à titre de principe actif, au moins l'un des composés ou sels pharmaceutiquement acceptables mentionnés dans la revendication 3.

5. Utilisation d'un des composés ou sels pharmaceutiquement acceptables mentionnés dans la revendication 3 pour préparer un médicament destiné à traiter des désordres / pathologies choisis parmi les maladies neurodégénératives, la douleur et l'épilepsie.

6. Utilisation selon la revendication 5, **caractérisée en ce que** le médicament préparé est destiné à traiter les maladies neurodégénératives.

7. Utilisation selon la revendication 6, **caractérisée en ce que** le composé ou sel pharmaceutiquement acceptable utilisé est choisi parmi le 4-[2-(1-aminocyclopentyl)-1,3-thiazol-4-yl]-2,6-di-tert-butylphénol et ses sels pharmaceutiquement acceptables.

8. Utilisation selon la revendication 7, **caractérisée en ce que** le médicament préparé est destiné à traiter la douleur.

## Patentansprüche

1. Eine Verbindung, ausgewählt aus den folgenden Verbindungen:
- 2,6-Di-*tert*-butyl-4-{2-[3-methyl-1-(methylamino)butyl]-1,3-thiazol-4-yl}phenol,
- 2,6-Di-*tert*-butyl-4-{2-[(1*S*)-1-(methylamino)ethyl]-1,3-thiazol-4-yl}phenol,
- 2,6-Di-*tert*-butyl-4-{2-[(1*R*)-1-(methylamino)ethyl]-1,3-thiazol-4-yl}phenol,
- 4-{2-[(1*R*)-1-Aminoethyl]-1,3-thiazol-4-yl}-2,6-di-*tert*-butylphenol,
- 4-{2-[(1*S*)-1-Aminoethyl]-1,3-thiazol-4-yl}-2,6-di-*tert*-butylphenol,
- 4-[2-(1-Aminocyclopropyl)-1,3-thiazol-4-yl]-2,6-di-*tert*-butylphenol,
- 4-[2-(1-Aminocyclopentyl)-1,3-thiazol-4-yl]-2,6-di-tert-butylphenol,
oder ein Salz davon.

2. Die Verbindung oder das Salz nach Anspruch 1, **dadurch gekennzeichnet, dass** es sich um 4-[2-(1-Aminocyclopentyl)-1,3-thiazol-4-yl]-2,6-di-*tert*-butylphenol oder eines seiner Salze handelt.

3. Eine Verbindung, ausgewählt aus den folgenden Verbindungen:
- 2 ,6-Di-*tert*-butyl-4-{2-[3-methyl-1-(methylamino)butyl]-1,3-thiazol-4-yl}phenol,
- 2,6-Di-*tert*-butyl-4-{2-[(1*S*)-1-(methylamino)ethyl]-1,3-thiazol-4-yl}phenol,
- 2,6-Di-*tert*-butyl-4-{2-[(1*R*)-1-(methylamino)ethyl]-1,3-thiazol-4-yl}phenol,
- 4-{2-[(1*R*)-1-Aminoethyl]-1,3-thiazol-4-yl}-2,6-di-*tert*-butylphenol,
- 4-{2-[(1*S*)-1-Aminoethyl]-1,3-thiazol-4-yl}-2,6-di-*tert*-butylphenol,
- 4-[2-(1-Aminocyclopropyl)-1,3-thiazol-4-yl]-2,6-di-*tert*-butylphenol,
- 4-[2-(1-Aminocyclopentyl)-1,3-thiazol-4-yl]-2,6-di-tert-butylphenol,
oder ein pharmazeutisch annehmbares Salz davon zur Verwendung als Medikament.

4. Eine pharmazeutische Zusammensetzung, die als Wirkstoff wenigstens eine der Verbindungen oder eines der pharmazeutisch annehmbaren Salze gemäß Anspruch 3 enthält.

5. Verwendung einer der Verbindungen oder eines der pharmazeutisch annehmbaren Salze gemäß Anspruch 3 zur Herstellung eines Medikaments zur Behandlung von Störungen / Erkrankungen, ausgewählt aus neurodegenerativen Erkrankungen, Schmerz und Epilepsie.

6. Verwendung nach Anspruch 5, **dadurch gekennzeichnet, dass** das hergestellte Medikament für die Behandlung von neurodegenerativen Erkrankungen bestimmt ist.

7. Verwendung nach Anspruch 6, **dadurch gekennzeichnet, dass** die Verbindung oder das pharmazeutisch annehmbare Salz ausgewählt ist aus 4-[2-(1-Aminocyclopentyl)-1,3-thiazol-4-yl]-2,6-di-tert-butylphenol und dessen pharmazeutisch annehmbaren Salzen.

8. Verwendung nach Anspruch 7, **dadurch gekennzeichnet, dass** das hergestellte Medikament für die Behandlung von Schmerz bestimmt ist.

## Claims

1. Compound chosen from the following compounds:
- 2,6-di-*tert*-butyl-4- {2-[3-methyl-1-(methylamino)butyl]-1,3-thiazol-4-yl}phenol;
- 2,6-di-*tert*-butyl-4- {2-[(1S)-1-(methylamino)ethyl]-1,3-thiazol-4-yl}phenol;
- 2,6-di-*tert*-butyl-4-(2-[(*1R*)-1-(methylamino)ethyl]-1,3-thiazol-4-yl}phenol;
- 4- {2-[(*1R*)-1-aminoethyl]-1,3-thiazol-4-yl}-2,6-di-*tert*-butylphenol ;
- 4- {2-[(*1S*)-1-aminoethyl]-1,3-thiazol-4-yl} -2,6-di-*tert*-butylphenol;
- 4-[2-(1-aminocyclopropyl)-1,3-thiazol-4-yl]-2,6-di-*tert*-butylphenol ;
- 4-[2-(1-aminocyclopentyl)-1,3-thiazol-4-yl]-2,6-di-*tert*-butylphenol ;
or a salt thereof.

2. Compound or salt according to claim 1, **characterized in that** it is the 4-[2-(1-aminocyclopentyl)-1,3-thiazol-4-yl]-2,6-di-*tert*-butylphenol or a salt thereof.

3. Compound chosen from the following compounds:
- 2,6-di-*tert*-butyl-4-{2-[3-methyl-1-(methylamino)butyl]-1,3-thiazol-4-yl}phenol;
- 2,6-di-*tert*-butyl-4-{2-[(1S)-1-(methylamino)ethyl]-1,3-thiazol-4-yl}phenol;
- 2,6-di-*tert*-butyl-4-(2-[(*1R*)-1-(methylamino)ethyl]-1,3-thiazol-4-yl}phenol;
- 4- {2-[(*1R*)-1-aminoethyl]-1,3-thiazol-4-yl}-2,6-di-*tert*-butylphenol ;
- 4- {2-[(*1S*)-1-aminoethyl]-1,3-thiazol-4-yl}-2,6-di-*tert*-butylphenol ;
- 4-[2-(1-aminocyclopropyl)-1,3-thiazol-4-yl]-2,6-di-*tert*-butylphenol ;
- 4-[2-(1-aminocyclopentyl)-1,3-thiazol-4-yl]-2,6-di-*tert*-butylphenol ;
or a pharmaceutically acceptable salt thereof for its use as a medicine.

4. Pharmaceutical composition containing, as active ingredient, at least one of the compounds or pharmaceutically acceptable salts mentioned in claim 3.

5. Use of one of the compounds or pharmaceutically acceptable salts mentioned in claim 3 to prepare a medicine intended for the treatement of disorders/pathologies chosen from the neurodegenerative diseases, pain and epilepsy.

6. Use according to claim 5, **characterized in that** the prepared medicine is intented for the treatment of neurodegenerative diseases.

7. Use according to claim 6, **characterized in that** the used compound or pharmaceutically acceptable salt is chosen from the 4-[2-(1-aminocyclopentyl)-1,3-thiazol-4-yl]-2,6-di-tert-butylphenol and its pharmaceutically acceptable salts.

8. Use according to claim 7, **characterized in that** the prepared medicine is intented for the treatment of pain.
